# EUROPÄISCHE PATENTANMELDUNG

(11) **EP 4 755 412 A1**
(43) Veröffentlichungstag der Anmeldung: **10.06.2026**
(21) Anmeldenummer: 24217232.8
(22) Anmeldetag: 03.12.2024
(51) Int. Cl.: A61M 3/02, A61H 35/04, A61J 1/06

(54) **NASENSPÜLGERÄT UND VERFAHREN ZU DESSEN HERSTELLUNG**

(71) Anmelder: Aptar Radolfzell GmbH, 78315 Radolfzell (DE)
(72) Erfinder: GREINER-PERTH, Jürgen, 78244 Gottmadingen (DE)
(74) Vertreter: Witte, Weller und Partner Patentanwälte mbB Stuttgart

(57) **Zusammenfassung**

Bekannt ist ein Nasenspülgerät (10) mit einem Flüssigkeitsspeicher (20) und einem Nasalapplikator (40), wobei der Nasalapplikator zur umlaufend abdichtenden Einführung in ein Nasenloch eines Benutzers ausgebildet ist und der Abgabe der Flüssigkeit aus dem Flüssigkeitsspeicher (20) in ein Nasenloch des Benutzers dient.

Vorgeschlagen wird, dass das Nasenspülgerät (10) einen einstückigen Hauptkörper (14) aufweist, der sowohl den Flüssigkeitsspeicher (20) als auch den Nasalapplikator (40) bildet. Der Hauptkörper (14) umschließt im Lieferzustand die Nasenspülflüssigkeit (12) vollumfänglich, wobei die Abgabeöffnung (42) im Lieferzustand durch einen werkzeuglos entfernbaren Verschluss (50) verschlossen ist, der ebenfalls Teil des einstückigen Hauptkörpers (14) ist.

## Beschreibung

### ANWENDUNGSGEBIET UND STAND DER TECHNIK

Die Erfindung betrifft ein Nasenspülgerät, das für die hygienische Reinigung und Pflege der Nasenhöhlen vorgesehen ist, sowie ein Verfahren zu dessen Herstellung.

Gattungsgemäße Nasenspülgeräte sind aus dem Stand der Technik bekannt und haben sich bewährt für die Entfernung von Schleim, Pollen, Staub oder anderen Partikeln, die die Atmung beeinträchtigen können. Die Verwendung von gattungsgemäßen Nasenspülgeräten geht üblicherweise damit einher, dass der Benutzer Flüssigkeit aus dem Nasenspülgerät unter geringem Druck in eines seiner Nasenlöcher einströmen lässt. Die Flüssigkeit füllt die Nasenhöhlen und tritt durch das andere Nasenloch wieder aus. Der Vorgang der Nasenspülung findet üblicherweise über einem Waschbecken statt, damit die aus dem anderen Nasenloch ausströmende Flüssigkeit direkt in das Waschbecken fließt.

Gattungsgemäße Nasenspülgeräte umfassen einen Flüssigkeitsspeicher und einen Nasalapplikator mit Abgabeöffnung, der die Abgabe einer Nasenspülflüssigkeit in Form eines unzerstäubten Flüssigkeitsstroms in das Nasenloch ermöglicht. Dabei dichtet der Nasalapplikator dieses Nasenloch gegenüber der Umgebung ab, so dass keine Flüssigkeit direkt entweichen kann. Weiterhin ist bei gattungsgemäßen Nasenspülgeräten vorgesehen, dass der Flüssigkeitsspeicher und der Nasalapplikator derart miteinander verbunden sind, dass sie im Wesentlichen in ortsfester Lage zueinander verbleiben. Dies ermöglicht es dem Benutzer, mit einer Hand den Flüssigkeitsspeicher zu führen und hierüber mittelbar den Nasalapplikator gegen die Wandung des Nasenlochs zu drücken, um die gewünschte Abdichtung zu erzielen.

Nasenspülgeräte der beschriebenen Art gibt es als Einweg-Produkte und zur Nachbefüllung. Bekannte Einweg-Produkte sind vergleichsweise teuer in der Herstellung. Zur Wiederverwendung und Nachbefüllung vorgesehene Nasenspülgeräte gehen mit der Gefahr einer Kontamination der Nasenspülflüssigkeit vor der Verwendung einher.

Wenngleich es im Stand der Technik schon eine Vielzahl von Lösungen gibt, besteht bei gattungsgemäßen Nasenspülgeräten noch Verbesserungsbedarf. Insbesondere ist eine einfache und kostengünstige Bauweise wünschenswert, die eine Gestaltung als Einwegartikel mit geringen Produktionskosten und guter Recyclingfähigkeit ermöglicht.

### AUFGABE UND LÖSUNG

Aufgabe der Erfindung ist es, ein Nasenspülgerät zur Verfügung zu stellen, welches gute Verwendbarkeit mit geringen Herstellungskosten und guter Recyclingfähigkeit verbindet.

Erfindungsgemäß wird ein Nasenspülgerät vorgeschlagen, welches in gattungsüblicher Art einen Flüssigkeitsspeicher und einen damit kommunizierend verbundenen Nasalapplikator aufweist.

Der Flüssigkeitsspeicher beinhaltet eine Flüssigkeitsmenge, die für die Spülung der Nase üblicherweise ausreicht. Insbesondere kann das Nasenspülgerät in unterschiedlichen Größen für Kinder und Erwachsene angeboten werden. Im Fall eines Nasenspülgeräts für Kinder ist der Flüssigkeitsspeicher vorzugsweise mit einem Flüssigkeitsvolumen von mindestens 20 ml befüllt, insbesondere zwischen 20 ml und 50 ml. Im Falle eines Nasenspülgeräts für Erwachsene beträgt das enthaltene Flüssigkeitsvolumen vorzugsweise mindestens 30 ml und insbesondere zwischen 30 ml und 80 ml. Das Volumen ist prinzipiell so zu wählen, dass es mindestens so groß ist wie das übliche Volumen der Nasenhöhlen der jeweiligen Zielgröße, insbesondere das durchschnittliche Volumen der Nasenhöhle der jeweiligen Zielgröße um mindestens 50 % übersteigt, so dass im Zuge der Entleerung Flüssigkeit bereits wieder durch das zweite Nasenloch austritt, während noch Flüssigkeit dem ersten Nasenloch zugeführt wird. Als besonders vorteilhaft wird es angesehen, wenn mindestens 40 ml, insbesondere mindestens 50 ml Nasenspülflüssigkeit im Flüssigkeitsspeicher gelagert sind.

Bei der Nasenspülflüssigkeit kann es sich insbesondere um eine medizinische oder hygienische Nasenspüllösung handeln. Insbesondere kommen nasale Salinelösungen, isotonische Kochsalzlösungen, Mentollösungen und Salbeilösungen in Frage. Zusätzlich können auch Lösungen mit entzündungshemmenden, pflegenden oder desinfizierenden Wirkstoffen verwendet werden, wie beispielsweise Kamille-Extrakte, Hyaluronsäure, Eukalyptusöl oder antiseptische Zusätze wie Chlorhexidin.

Darüber hinaus können physiologische Kochsalzlösungen verwendet werden, die Vorzugswqeise 0,9% NaCl enthält, sowie hypertone Kochsalzlösungen mit einem höheren NaCl-Gehalt, beispielsweise 3%, eingesetzt werden. Letztere sind geeignet, Wasser aus Gewebe ziehen und so eine abschwellende Wirkung entfalten kann. Angereicherte Kochsalzlösungen, die neben NaCl zusätzliche Inhaltsstoffe wie Zink, Selen, Dexpanthenol oder pflanzliche Extrakte wie Kamille oder Aloe vera enthalten, bieten zusätzliche Vorteile für die Nasenschleimhaut. Zudem können antivirale Präparate mit Inhaltsstoffen wie Carragelose verwendet werden, die Viren in den oberen Atemwegen binden und so die Besiedlung der Schleimhautzellen sowie die virale Replikation verhindern. Schließlich kommen auch Präparate mit direkter antiviraler oder desinfizierender Wirkung, wie Nasensprays auf Basis von Povidon-Iod, in Frage.

Grundsätzlich bevorzugt ist es, wenn die Nasenspülflüssigkeit konservierungsmittelfrei ist, um die Verträglichkeit zu gewährleisten, insbesondere für Allergiker oder Personen mit empfindlicher Nasenschleimhaut. Sofern ein steriler Abfüllvorgang gewährleistet wird, wird die Aufrechterhaltung der Sterilität bei einem erfindungsgemäßen Nasenspülgerät durch die vollständige Umhüllung durch den nachfolgend noch beschriebenen Hauptkörper im Lagerzeitraum sicher gewährleistet, so dass auf Konservierungsmittel verzichtet werden kann.

Der Nasalapplikator wird im Zuge der Verwendung bestimmungsgemäß in ein Nasenloch des Benutzers eingeschoben, bevor mit der Abgabe von Flüssigkeit begonnen wird. Der Nasalapplikator ist derart ausgebildet, dass er umlaufend an der Wandung des Nasenlochs anliegt, so dass keine Flüssigkeit am Applikator vorbei direkt aus dem Nasenloch auslaufen kann.

Am distalen Ende des Nasalapplikators ist eine Abgabeöffnung zur Abgabe eines unzerstäubten Flüssigkeitsstroms von Flüssigkeit aus dem Flüssigkeitsspeicher vorgesehen. Diese Abgabeöffnung ist vorzugsweise vergleichsweise groß gestaltet, um einen ungestörten Flüssigkeitsstrom zu ermöglichen. Grundsätzlich sind auch Bauweisen mit mehreren Abgabeöffnung möglich. Bevorzugt wird jedoch eine einzige Abgabeöffnung, nach Möglichkeit mit einem geringen Strömungswiderstand.

Insbesondere weist der Nasalapplikator vorzugsweise eine sich zu seinem Ende und der dort vorgesehenen Abgabeöffnung hin verjüngende Form auf, so dass der Benutzer den Nasalapplikator in das Nasenloch einführen kann, bis er schließlich umlaufend anliegt. Insbesondere kann der Nasalapplikator abschnittsweise mit einem konischen Kontaktabschnitt zur Anlage an eine Nasenlochwandung des Benutzers ausgebildet sein, um für verschiedene Benutzer gut zu passen. Im Falle eines solchen konischen Kontaktabschnitts an einem Nasenspülgerät für Erwachsene weist die durchmessergrößte Stelle des Kontaktabschnitts vorzugsweise einen Außendurchmesser von mindestens 15 mm auf. Bei einem Nasenspülgerät für Kinder weist der Kontaktabschnitt vorzugsweise an der durchmessergrößten Stelle einen Außendurchmesser von mindestens 8 mm, insbesondere von mindestens 10 mm, auf.

Der Nasalapplikator und der Flüssigkeitsspeicher sind jeweils als starre oder elastisch verformbare Teilkörper ausgebildet und miteinander derart verbunden, dass sie eine im Wesentlichen feste Relativstellung zueinander einnehmen können. Wenngleich der Flüssigkeitsspeicher je nach Ausgestaltung bestimmungsgemäß zusammengedrückt wird, ist er ausreichend starr, um mittelbar über Führen des Flüssigkeitsspeichers auch das Führen des Nasalapplikators und das abdichtende Andrücken des Nasalapplikators an die Nase zu gestatten.

Der Benutzer kann das Nasenspülgerätführen, indem er den Flüssigkeitsspeicher ergreift und durch dessen Bewegung auch den Nasalapplikatorführt und in das Nasenloch einschieben kann. Während des Austritts der Nasenspülflüssigkeit durch die Auslassöffnung kann der Benutzer den Nasalapplikator dauerhaft gegen die Nase drücken, um dort die Dichtigkeit zu gewährleisten.

Der Nasalapplikator kann unmittelbar am Flüssigkeitsspeicher angrenzen, aber auch mittels eines Verbindungsstücks mit dem Flüssigkeitsspeicher verbunden sein. Ein solches Verbindungsstück kann insbesondere eine Biegung aufweisen, durch die der Nasalapplikator in eine für die Flüssigkeitsabgabe geeignete Richtung weist, obwohl das proximale Ende des Verbindungsstücks in eine andere Richtung weisend am Flüssigkeitsspeicher angebracht ist.

Das Verbindungsstück muss keine vollständig unveränderbare Relativposition des Nasalapplikators gegenüber dem Flüssigkeitsspeicher definieren. Auch möglich ist eine Gestaltung, bei der das Verbindungsstück in unterschiedliche Positionen relativzum Flüssigkeitsspeicher verbracht werden kann, die dann selbsttätig gehalten werden. Erzielbar ist so etwas bspw. mit einem abschnittsweise in Form eines stabilen Balges ausgebildeten Verbindungsstücks. Insbesondere bevorzugt ist jedoch eine Gestaltung, bei der eine solche Beweglichkeit nicht gegeben ist und der Flüssigkeitsspeicher und der Nasalapplikator stets in fester Relativlage zueinander verbleiben.

Das erfindungsgemäß vorgeschlagene Nasenspülgerät zeichnet sich durch einen einstückigen Hauptkörper aus, der sowohl den Flüssigkeitsspeicher als auch den Nasalapplikator umfasst. Insbesondere handelt es sich um einen einstückigen Hauptkörper aus Kunststoff.

Unter einer solchen einstückigen Gestaltung des Hauptkörpers und des Nasalapplikators wird verstanden, dass der Hauptkörper und der Nasalapplikator sowie ggf. auch das Verbindungsstück unmittelbar stoffschlüssig miteinander verbunden sind. Vorzugsweise ist die Grundform des Hauptkörpers mittels Blasformen hergestellt, also indem ein Rohling innerhalb einer Form druckluftbeaufschlagt wird und sich hierdurch an eine umgebende formgebende Wandung anlegt.

Der Hauptkörper umschließt im Lieferzustand nicht nur die Nasenspülflüssigkeit im Flüssigkeitsspeicher sowie im Nasalapplikator, sondern er bildet auch einen werkzeuglos entfernbaren Verschluss, der im Lieferzustand die Abgabeöffnung verschließt und der ebenfalls Teil des einstückigen Hauptkörpers ist.

Grundsätzlich sind zwei Arten von erfindungsgemäßen Nasenspülgeräten möglich, nämlich Nasenspülgeräte, deren Flüssigkeit bei richtiger Ausrichtung und Vorbereitung schwerkraftbedingt in die Nase des Benutzers einströmt, sowie Nasenspülgeräte, bei denen die Flüssigkeit im Flüssigkeitsspeicher druckbeaufschlagt wird und bedingt oder unterstützt durch den manuell aufgebrachten Druck in Richtung der Abgabeöffnung strömt und hieraus abgegeben wird. Insbesondere kann hierfür vorgesehen sein, dass der Flüssigkeitsspeicher mit einer Wandungsstärke ausgestattet ist, die ein Zusammendrücken in Form einer elastisch verformbaren Quetschflasche gestattet.

Im Falle einer Gestaltung, bei der die Nasenspülflüssigkeit gravitativ bedingt abgegeben wird, wird es als vorteilhaft angesehen, wenn das Nasenspülgerät zusätzlich zur Abgabeöffnung eine Lufteinlassöffnung aufweist, so dass während der Abgabe von Flüssigkeit Luft in den Flüssigkeitsspeicher nachströmen kann. Während die Abgabeöffnung an einem tiefen oder dem tiefsten Punkt des Flüssigkeitsspeichers vorgesehen ist, ist die Lufteinlassöffnung gegenüberliegend und vorzugsweise am höchsten Punkt des Nasenspülgeräts bezogen auf dessen bestimmungsgemäße Verwendungsstellung angeordnet.

Vorzugsweise ist die genannte Lufteinlassöffnung im Lieferzustand ebenso wie die Abgabeöffnung durch einen werkzeuglos entfernbaren zweiten Verschluss verschlossen, der ebenfalls Teil des einstückigen Hauptkörpers ist.

Der erste Verschluss an der Abgabeöffnung und ggf. der zweite Verschluss an der Lufteinlassöffnung sind für ein werkzeugloses Öffnen ausgebildet, wobei dies meist bedeutet, dass die jeweiligen Öffnungen durch den Benutzer ohne zusätzliches Werkzeug, welches nicht Teil des Nasenspülgeräts ist, geöffnet werden können, wobei es hierdurch zur Trennung des Materials des Hauptkörpers an einer den Verschluss umgebenden Trennkante kommt. Insbesondere soll es einem erwachsenen Benutzer möglich sein, den mindestens einen Verschluss mit den Fingern zu öffnen, auch wenn der Benutzer nicht über große Körperkraft verfügt. Es kann vorgesehen sein, dass der jeweilige Verschluss bestimmungsgemäß gedreht wird, um die Abgabeöffnung und vorzugsweise auch die Lufteinlassöffnung freizugeben. Es kann sich aber beispielsweise auch um eine Abreißlasche handeln, die stoffschlüssig und aus einheitlichem Material zum restlichen Hauptkörper an der jeweiligen Öffnung angebracht ist.

Vorzugsweise verfügt der jeweilige Verschluss über einen Griffabschnitt, wobei durch eine Relativbewegung des Verschlusses gegenüber dem Flüssigkeitsspeicher bzw. dem Nasalapplikator mittels dieses Griffabschnitts der Verschluss werkzeuglos abgetrennt werden kann. Der Griffabschnitt ist vorzugsweise mit Greifflächen vorgesehen, die ausreichend groß sind, um auch von einem Erwachsenen ergriffen zu werden. Insbesondere kann der Griffabschnitt in Form einer dünnen Kunststoffplatte oder eines Kunststoffstegs ausgebildet sein, wobei die Platte oder der Steg vorzugsweise die mittlere Abgaberichtung bzw. die mittlere Einströmrichtung kreuzt, so dass der Verschluss mittels der Platte oder des Stegs um diese Richtungsachse gedreht werden kann, um hierdurch den Verschluss vom Hauptkörper zu trennen. Im Falle einer Abreißlösung bildet der Griffabschnitt vorzugsweise eine Lasche, die bei Kraftbeaufschlagung in definierter Richtung ein sicheres umlaufendes Trennen des Verschlusses von der Öffnung gestattet.

Die Verschlüsse können nach dem Öffnen weggeworfen werden, sofern sie nicht mittels eines Haltesteges zum dauerhaften Verbleib am Hauptkörper vorgesehen sind. Üblicherweise sind Nasenspülgeräte der erfindungsgemäßen Art nicht zur Wiederverwendung vorgesehen, so dass die Verschlüsse nach dem Öffnen nicht mehr benötigt werden. Es sind jedoch auch Bauweisen denkbar und von der Erfindung umfasst, bei der mindestens ein Verschluss als wiederverwendbarer Verschluss ausgebildet ist. Der Verschluss kann insbesondere derart ausgebildet sein, dass er im Lieferzustand die entsprechende Öffnung verschließend einstückiger Teil des Hauptkörpers ist und nach Abtrennen hiervon und damit nach Öffnen der Öffnung in einer anderen Ausrichtung wieder aufsetzbar ist, um die Öffnung wieder zu verschließen, beispielsweise indem ein am Verschluss vorgesehenes Innengewinde auf ein Außengewinde des Nasalapplikators aufgeschraubt wird.

Beim Abtrennen der Verschlüsse werden Wandungsabschnitte an der Abgabeöffnung bzw. der Lufteinlassöffnung bestimmungsgemäß zerstörungsbehaftet getrennt. Dies kann zu einer Scharfkantigkeit an der Bruchstelle führen, welche in beiden Fällen störend sein kann. Im Falle der Abgabeöffnung kann die Scharfkantigkeit die Nase verletzen. Im Fall der Lufteinlassöffnung kann die Scharfkantigkeit an den Fingern schmerzen, wenn die Lufteinlassöffnung während der Verwendung phasenweise zugehalten wird.

Es kann daher von Vorteil sein, wenn der erste Verschluss der Abgabeöffnung und/oder der zweite Verschluss der Lufteinlassöffnung derart ausgebildet sind, dass die Trennkante am Hauptkörper, die nach Entfernen des Verschlusses verbleibt, durch einen Schutzrand umgeben ist. Dies bedeutet, dass die Sollreißlinie gegenüber diesem Schutzrand zurückgesetzt ist. Im Falle der Abgabeöffnung ist der Schutzrand vorzugsweise zwischen 0,5 mm und 2 mm von der Mittelachse der Abgabeöffnung beabstandet. Im Falle der Lufteinlassöffnung ist der Schutzrand vorzugsweise zwischen 3 mm und 12 mm von der Mittelachse der Lufteinlassöffnung beabstandet.

Im Falle der Lufteinlassöffnung übernimmt dieser Schutzrand vorzugsweise auch die Abdichtung am Finger des Benutzers, wenn phasenweise das Einströmen von Luft - und damit mittelbar das Ausströmen von Flüssigkeit - durch Zudrücken der Lufteinlassöffnung unterbrochen wird.

Der Hauptkörper, der den Flüssigkeitsspeicher umgibt und den Nasalapplikator bildet, ist vorzugsweise als einheitlicher Kunststoffkörper ausgebildet. Insbesondere kann vorgesehen sein, dass dieser Kunststoffkörper über eine umgebende Wandung mit einer Wandstärke zwischen 0,3 mm und 1,0 mm verfügt. Sofern der Flüssigkeitsspeicher einen elastisch verformbaren Quetschabschnitt bildet, der bestimmungsgemäß zur Abgabe der Flüssigkeit zusammengedrückt wird, beträgt die Wandstärke zumindest im Quetschabschnitt vorzugsweise zwischen 0,3 mm und 0,8 mm, insbesondere zwischen 0,3 mm und 0,6 mm. Sofern der Flüssigkeitsspeicher bestimmungsgemäß nicht zusammengedrückt wird, sondern die Abgabe von Flüssigkeit stattdessen schwerkraftbedingt erfolgt, während Luft durch eine Lufteinlassöffnung nachströmen kann, wird eine größere Wandstärke des Flüssigkeitsspeichers bevorzugt, bevorzugt zwischen 0,6 mm und 1,0 mm.

Als Herstellungstechnik für den Hauptkörper findet insbesondere das Blasformen von Kunststoffen Verwendung. Blasformen ist ein Fertigungsverfahren, bei dem ein Kunststoff in Form eines Rohlings durch Druckluft in eine äußere Form geblasen wird, um Hohlkörper wie den hier benötigten Hauptkörper herzustellen. Hierdurch kann dem Hauptkörper seine Grundform gegeben werden, wobei vorzugsweise zum Formen des mindestens einen Verschlusses nach Abschluss des Blasformens ein separater thermischer Umformungsvorgang vorgesehen ist.

Da es beim Blasformen recht aufwändig ist, die Wandungsdicke abschnittsweise anzupassen, wird es als bevorzugt angesehen, wenn die Wandungen des Hauptkörpers mit Ausnahme der Bereiche der Verschlüsse eine einheitliche Wandstärke aufweisen. Dies bedeutet, dass die Wandstärke um nicht mehr als 20 % variiert. Es kann aber durchaus vorteilhaft sein, variable Wandungsstärken, ggf. auch mit Variationen oberhalb der genannten 20%, vorzusehen, um den einstückigen Hauptköper partiell besonders zu gestalten, im Bereich eines Quetschabschnitte jedoch eine erleichterte Beweglichkeit vorzusehen.

Der Hauptkörper besteht bei einem erfindungsgemäßen Nasenspülgerät vorzugsweise aus Kunststoff. Dabei haben sich Polyethylen, Polypropylen, Cycloolefin-Copolymere, Polyethylenterephthalat, Ethyl-Vinylalkohol, Polyvinylchlorid und Polypropylen-Copolymere als gut verwendbar herausgestellt. Polyethylen (PE) eignet sich gut, da es leicht, chemikalienbeständig und flexibel ist. Polypropylen (PP) bietet eine ausgezeichnete Wärmebeständigkeit und Festigkeit, was besonders für sterilisierbare Nasenspülgeräte vorteilhaft ist. Cycloolefin-Copolymere (COC) überzeugen durch ihre hohe Transparenz und eine ausgezeichnete Feuchtigkeitsbarriere, was die Haltbarkeit und die optische Kontrolle des Inhalts begünstigt. Polyethylenterephthalat (PET) ist besonders dann sinnvoll, wenn eine hohe Stabilität erzielt werden muss, insbesondere im Falle von Nasenspülgeräten mit Lufteinlassöffnung, die bestimmungsgemäß nicht zusammengedrückt werden. Es weist eine hohe Festigkeit und Stabilität auf, wodurch Nasenspülgeräte sowohl leicht als auch bruchsicher gestaltet werden können. Ethyl-Vinylalkohol (EVOH) zeichnet sich durch seine außergewöhnliche Gasbarriere aus und schützt die Nasenspüllösung effektiv vor Sauerstoffeintritt, was die Haltbarkeit verlängert. Polyvinylchlorid (PVC) bietet eine gute chemische Beständigkeit und lässt sich leicht verarbeiten, wodurch es sich für Hauptkörper von Nasenspülgeräten mit komplexeren Formen eignet. Polypropylen-Copolymere (PP-C) kombinieren Flexibilität mit Festigkeit und ermöglichen Nasenspülgeräte, die sowohl langlebig als auch angenehm in der Handhabung sind.

Zweckmäßig kann es auch sein, wenn der Hauptkörper vollständig oder abschnittsweise transparent ausgebildet ist, um die im Hauptkörper verbleibende Flüssigkeitsmenge für den Benutzer erkennbar zu machen.

Als bevorzugt wird es weiterhin angesehen, wenn der Flüssigkeitsspeicher und damit der entsprechende Abschnitt des Hauptkörpers eine zylindrische Grundform aufweist, insbesondere eine kreiszylindrische Grundform. Eine solche Grundform gestattet ein vergleichsweise kleines Gerät trotz großen Flüssigkeitsspeichers. Auch ist die Herstellung von abschnittsweise zylindrischen Körpern vorteilhaft, wenn als Fertigungstechnik das Kunststoff-Blasformen gewählt wird, was vorliegend die für den Hauptkörper empfohlene Fertigungstechnik ist.

Im Falle eines zylindrischen und insbesondere kreiszylindrischen Flüssigkeitsspeicherabschnitts des Hauptkörpers ist es von Vorteil, wenn an einem oberen Ende des Flüssigkeitsspeichers die Lufteinlassöffnung vorgesehen ist, insbesondere fluchtend mit einer Mittelachse der Grundform. Demgegenüberliegend ist vorzugsweise an einem unteren Ende des Flüssigkeitsspeichers der Nasalapplikator oder das zum Nasalapplikator führende Verbindungsstück vorgesehen, insbesondere auch in diesem Falle fluchtend mit einer Mittelachse.

Im Falle eines Flüssigkeitsspeichers, der durch einen kreiszylindrischen Abschnitt des Hauptkörpers gebildet ist, wird es als bevorzugt angesehen, wenn die kreiszylindrische Grundform im Bereich des Flüssigkeitsspeichers mit einem Außendurchmesser zwischen 20 mm und 60 mm versehen ist, vorzugsweise mit einem Außendurchmesser zwischen 25 mm und 40 mm.

Es kann sinnvoll sein, von der kreiszylindrischen Grundform abzuweichen, um das Handling zu erleichtern. Insbesondere kann es sinnvoll sein, eine andere Formgebung im Bereich des Flüssigkeitsspeichers zu wählen, so dass der Benutzer anhand dessen die Ausrichtung des Nasalapplikators abschätzen kann und diesen damit ohne genaueres Hinzuschauen in ein Nasenloch einschieben kann. Eine Möglichkeit zur Erzielung dessen liegt in einem Flüssigkeitsspeicher mit einem elliptischen Querschnitt.

Eine weitere bevorzugte Gestaltung sieht vor, dass der Flüssigkeitsspeicher zumindest abschnittsweise einen Querschnitt mit zueinander nicht-parallelen planen Wandungsabschnitten aufweist, insbesondere einen im Wesentlichen dreieckigen, viereckigen, fünfeckigen oder sechseckigen Querschnitt. Diese Formgebungen haben sich ebenfalls als haptisch angenehm und praktisch für den Bediener herausgestellt. Insbesondere ist ein viereckiger Querschnitt denkbar, der bezogen auf die Flüssigkeitsmenge im Flüssigkeitsspeicher eine vergleichsweise kleine Umverpackung ermöglicht.

Sofern es sich um ein Nasenspülgerät handelt, welches primär für Kinder vorgesehen ist, kann es von Vorteil sein, eine für Kinder ästhetisch ansprechende Formgebung zu wählen. Insbesondere kann das Nasenspülgerät bzw. dessen Hauptkörper, die stilisierte Form eines Tieres aufweisen, wobei insbesondere ein Maul oder ein Rüssel des stilisierten Tieres den Nasalapplikator bildet. Insbesondere Gestaltungen des Hauptkörpers als stilisierter Fisch oder Delphin oder als stilisierter Elefant eignen sich gut für die hier vorgestellte Verwendung.

Ebenfalls zweckmäßig kann es sein, dem Hauptkörper im Bereich des Flüssigkeitsspeichers eine flache Formgebung zu verleihen. So kann der Flüssigkeitsspeicher insbesondere zumindest abschnittsweise einen flachen Querschnitt aufweisen, der in einer langen Erstreckungsrichtung eine Erstreckung aufweist, die mindestens den Faktor 1,5 so groß ist wie in einer kurzen Erstreckungsrichtung. Beispielsweise kann der Querschnitt eine Ellipsenform aufweisen.

Wie bereits geschildert, betrifft eine der möglichen Gestaltungen eines Nasenspülgeräts gemäß der Erfindung eine Gestaltung, bei der der Flüssigkeitsspeicher bestimmungsgemäß zusammengedrückt wird, um dadurch das Innenvolumen zu verringern und die Flüssigkeit in Richtung der Abgabeöffnung zu drücken.

Verschiedene Maßnahmen sind für diese Gestaltung zweckmäßig, so beispielsweise die angepassten Wandstärken, die oben erwähnt sind. So kann insbesondere vorgesehen sein, dass der Flüssigkeitsspeicher zumindest abschnittsweise mit einem Balgabschnitt ausgebildet ist, der die Reduzierung des Innenvolumens erleichtert. Ein Balgabschnitt in diesem Sinne zeichnet sich durch eine Abfolge von Wellenformen oder Faltenformen in der Wandung aus, die ein Zusammendrücken erleichtern, hierfür jedoch eine geringere Rückstellneigung als wellen- oder faltenfreie Wandungen aufweisen. Da es bei einem erfindungsgemäßen Nasenspülgerät üblicherweise nicht auf eine schnelle Rückstellung ankommt, ist insbesondere der Vorteil des durch die Balgform bedingten sehr leichtgängigen Zusammendrückens relevant. Diese Leichtgängigkeit gestattet eine besonders feine Dosierung.

Eine andere vorteilhafte Formgebung, die sich insbesondere dann eignet, wenn der Flüssigkeitsspeicher mit Quetschabschnitt ausgebildet ist, ist gegeben, wenn der Flüssigkeitsspeicher zwei einander gegenüberliegende Betätigungs-Wandabschnitte zum Zwecke der manuellen Krafteinkopplung aufweist. Hier legt der Benutzer bestimmungsgemäß seine Finger auf, um den Flüssigkeitsspeicher zu verkleinern. Die Betätigungs-Wandabschnitte weisen hierfür eine ausreichende Größe auf. Insbesondere kann vorgesehen sein, dass an mindestens einem der Betätigungs-Wandabschnitte eine vertiefte oder mit Greifstrukturierungen versehene Grifffläche vorgesehen ist, die die Handhabung vereinfacht.

Wie eingangs schon erläutert, weist ein erfindungsgemäßes Nasenspülgerät vorzugsweise ein Verbindungsstück auf, welches den Flüssigkeitsspeicher und den Nasalapplikator verbindet. Dieses Verbindungsstück muss nicht in gesonderter Weise abgesetzt sein, sondern kann sich als einheitlicher Rohrabschnitt zwischen dem Flüssigkeitsspeicher und dem Nasalapplikator darstellen, der ohne Absatz in den Nasalapplikator übergeht.

Das Verbindungsstück und der sich daran anschließende Nasalapplikator können in Richtung der Mittelachse des Flüssigkeitsspeichers erstreckt sein. Vorzugsweise weichen das Verbindungsstück und der Nasalapplikator jedoch von dieser Ausrichtung ab, um eine bequemere Handhabung zu ermöglichen.

Ein Verbindungsstück der genannten Art kann bei einer auch dem Zweck dienen, die Ausrichtung des Nasalapplikators an die gewünschte Handhabung des Nasenspülgerätes anzupassen, insbesondere indem das Verbindungsstück eine Anwinkelung des Nasalapplikators zum Flüssigkeitsspeicher bewirkt, vorzugsweise mit einem Winkel zwischen 60° und 120° zwischen einer Mittelachse der kreiszylindrischen Grundform und der durch die Abgabeöffnung definierten Abgaberichtung.

Das Verbindungsstück weist vorzugsweise einen Außendurchmesser auf, der dem größten Außendurchmesser am Nasalapplikator entspricht oder diesen übersteigt. Dies bedeutet, dass die Formgebung des Verbindungsstücks und des Nasalapplikators sich zum distalen Ende hin verjüngt, was die Herstellung vereinfachen kann.

Insbesondere jedoch kann vorgesehen sein, dass das Verbindungsstück einen Außendurchmesser aufweist, der kleiner ist als der größte Außendurchmesser am Nasalapplikator. Insbesondere kann der Nasalapplikator seinen größten Durchmesser im Bereich der schon beschriebenen konischen Kontaktfläche aufweisen, wobei gemäß dieser Gestaltung das sich daran Richtung Flüssigkeitsspeicher anschließende Verbindungsstück einen demgegenüber kleineren Außendurchmesser aufweist.

Wie schon beschrieben wurde, erfolgt die Fertigung des beschriebenen Nasenspülgeräts vorzugsweise mittels Blasformen. Insbesondere ist ein integrierter Prozess zweckmäßig, bei dem zunächst der Hauptkörper mittels Blasformen erstellt wird und unmittelbar anschließend die durch diese Art der Herstellung gewährleistete Sterilität genutzt wird, um unmittelbar Flüssigkeit in den Hauptkörper einzufügen und anschließend einen der Verschlüsse zu schließen. Im Falle einer Gestaltung mit zwei Verschlüssen ist ein erster Verschluss vorzugsweise schon ausgebildet und die zugehörige Öffnung geschlossen, wenn die Nasenspülflüssigkeit in den Hauptkörper eingefügt wird.

Grundsätzlich ist gewünscht, dass das Innenvolumen des Hauptkörpers gut ausgenutzt wird. Es ist daher zweckmäßig, wenn der Hauptkörper einschließlich des Innenvolumens des Verbindungsstücks und des Nasalapplikators zu mindestens 50 % mit Nasenspülflüssigkeit befüllt ist, vorzugsweise zu mindestens 60 %. Gleichzeitig ist es jedoch auch gewollt, dass ein Restvolumen nach Befüllung des Hauptkörpers und Schließen der Verschlüsse verbleibt, welches nicht mit Flüssigkeit befüllt wird. Dieses Restvolumen erleichtert die Handhabung - beispielsweise, weil es verhindert, dass nach dem Öffnen der Verschlüsse unmittelbar Flüssigkeit entweicht. Es ist insgesamt bevorzugt, dass ein Innenvolumen des Hauptkörpers zu maximal 90 % mit der Nasenspülflüssigkeit befüllt ist, vorzugsweise zu maximal 75 %.

Bei einer Gestaltung des Nasenspülgeräts, bei der die Flüssigkeit nicht durch eine Volumenverringerung des Flüssigkeitsspeichers herausgedrückt wird, sondern bei gleichzeitig nachströmender Luft schwerkraftbedingt durch die Abgabeöffnung abgegeben wird, ist darauf zu achten, dass der Hauptkörper und der Nasalapplikator so gestaltet sind, dass ein ausreichender Flüssigkeitsstrom für den Austrag gewährleistet ist. Hierfür ist es zunächst geboten, den Hauptkörper und den Nasalapplikator derart zu gestalten, dass der Flüssigkeitsspeicher während des Austrags oberhalb der Abgabeöffnung angeordnet ist. Vorzugsweise befindet sich der Flüssigkeitsspiegel vor Entnahme von Flüssigkeit bei tief angeordnetem Nasalapplikator mindestens 80 mm oberhalb der Abgabeöffnung, vorzugsweise mindestens 100 mm oberhalb der Abgabeöffnung. Gleichzeitig sind die Abgabeöffnung und der Zulauf zur Abgabeöffnung vorzugsweise derart ausgestaltet, dass sie nur einen geringen Strömungswiderstand verursachen. Die Abgabeöffnung weist vorzugsweise eine lichte Durchtrittsfläche von mindestens 3 mm² auf, insbesondere vorzugsweise von mindestens 6 mm² oder mindestens 10 mm². Vorzugsweise ist auch der Querschnitt eines Flüssigkeitskanals vom Flüssigkeitsspeicher zur Abgabeöffnung mit einem geringsten lichten Querschnitt von mindestens 3 mm² versehen, insbesondere vorzugsweise von mindestens 6 mm² oder mindestens 10 mm².

Die Erfindung betrifft neben dem Nasenspülgerät als solchem auch ein Verfahren zu dessen Herstellung. Vorgeschlagen wird ein Verfahren mit den folgenden Schritten:
Zunächst wird der Hauptkörper des Nasenspülgeräts, der sowohl den Flüssigkeitsspeicher als auch den Nasalapplikator bildet, in einer Formkavität als einstückige Einheit geformt, wobei zunächst mindestens eine Öffnung im Hauptkörper verbleibt. Dies erfolgt vorzugsweise durch Extrusion eines schlauchförmigen Rohlings aus der Kunststoffschmelze. Dieser Schlauch wird in die genannte Formkavität eingebracht und mit Druckluft aufgeblasen, so dass sich eine Formgebung des Kunststoffteils entsprechend der Formkavität ergibt (Blasformen).

Nach dieser Formung des Hauptkörpers, der aufgrund der Temperaturen bei der Herstellung steril ist, wird die Nasenspülflüssigkeit im ebenfalls sterilen Zustand durch die Öffnung in den Flüssigkeitsspeicher des Hauptkörpers eingefüllt, ohne dass der Hauptkörper hierfür die sterile Umgebung verlässt. Wie oben bereits erläutert, wird der Hauptkörper vorzugsweise zu mindestens 50 %, insbesondere zu mindestens 60 % mit Flüssigkeit befüllt. Der Füllgrad kann auch höher sein. Er sollte jedoch vorzugsweise nicht mehr als 95 %, vorzugsweise nicht mehr als 85 %, betragen, um das Verschließen des Hauptkörpers nicht zu erschweren.

Sobald die Befüllung abgeschlossen ist, erfolgt das Verschließen der Öffnung. Der Hauptkörper wird im Bereich der Öffnung unter Nutzung des Materials des Hauptkörpers verschlossen. Insbesondere erfolgt dies in Form einer thermischen und mechanischen Versiegelung. Dabei erfolgt vorzugsweise mittels einer Verschlusskavität auch die Formgebung des manuell und werkzeuglos abnehmbaren Verschlusses.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1A bis 1C zeigen ein erstes Ausführungsbeispiel eines Nasenspülgeräts.
Fig. 2A bis 2D verdeutlichen die Inbetriebnahme und Nutzung des Nasenspülgeräts der Fig. 1A bis 1C.
Fig. 3A bis 13 zeigen ein zweites Ausführungsbeispiel eines Nasenspülgeräts.
Fig. 4A bis 4D verdeutlichen die Inbetriebnahme und Nutzung des Nasenspülgeräts der Fig. 2A bis 2C.
Fig. 5A bis 5C zeigen ein drittes Ausführungsbeispiel eines Nasenspülgeräts.
Fig. 6A bis 6D zeigen ein viertes Ausführungsbeispiel eines Nasenspülgeräts.
Fig. 7A bis 7C zeigen ein fünftes Ausführungsbeispiel eines Nasenspülgeräts.
Fig. 8 bis 10 verdeutlichen mögliche Variationen des Querschnitts des Flüssigkeitsspeichers des Nasenspülgeräts.
Fig. 11A bis 11C zeigen ein sechstes Ausführungsbeispiel eines Nasenspülgeräts.
Fig. 12 bis 14 zeigen ein weitere Ausführungsbeispiele eines Nasenspülgeräts.
Fig. 15A und 15B zeigen ein weiteres Ausführungsbeispiel eines Nasenspülgeräts, in diesem Falle mit wiederwendbarem Verschluss.
Fig. 16A bis 16I verdeutlichen die Herstellung des Nasenspülgeräts der Fig. 1A bis 1C.

### DETAILLIERTE BESCHREIBUNG DER AUSFÜHRUNGSBEISPIELE

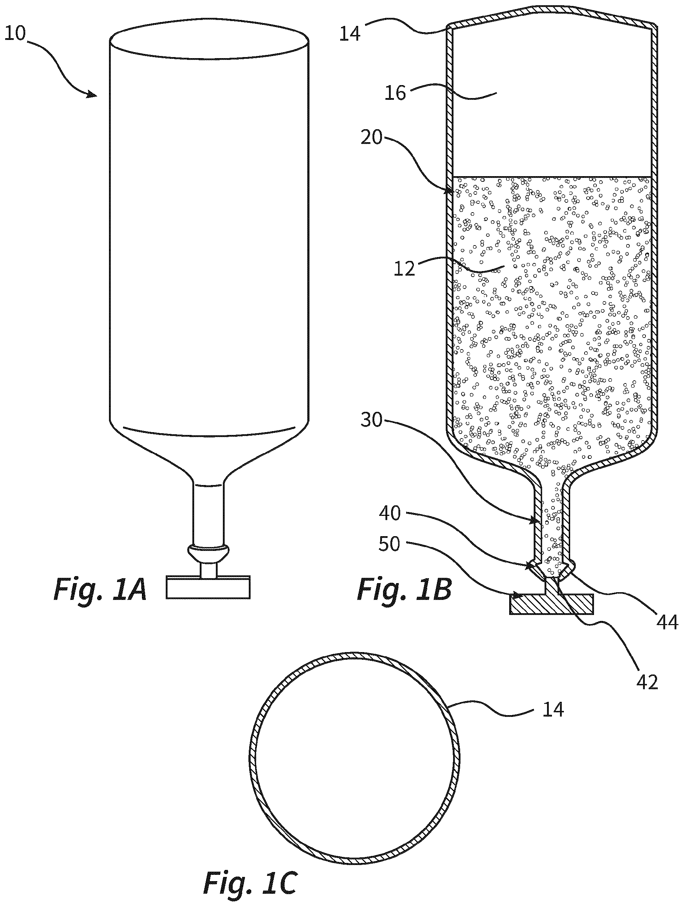

Fig. 1A bis 1C zeigen ein erstes Ausführungsbeispiel eines erfindungsgemäßen Nasenspülgeräts 10. Dabei zeigt Fig. 1A das Nasenspülgerät 10 von außen im Lieferzustand. Die Fig. 1B und 1C zeigen einen Längsschnitt und einen Querschnitt durch das Nasenspülgerät 10.

Das Nasenspülgerät 10 weist einen sehr einfachen Aufbau auf. Es verfügt über einen Hauptkörper 14 aus Kunststoff, der verschiedene Teile des Nasenspülgeräts 10 bildet, nämlich insbesondere die Wandungen eines Flüssigkeitsspeichers 20, einen Nasalapplikator 40 sowie ein Verbindungsstück30 dazwischen. Weiterhin bildet der Hauptkörper 14 im Lieferzustand auch einen Verschluss 50, mittels dessen eine Abgabeöffnung 42 am Nasalapplikator40 im Lieferzustand verschlossen ist.

Mit Ausnahme des Verschlusses 50 weist der Hauptkörper 14 eine rotationssymmetrische Gestaltung auf. Der Flüssigkeitsspeicher 20 weist eine kreiszylindrische Formgebung auf und wird am oberen Ende durch eine vorliegend nach außen gewölbte Wandung begrenzt. Am unteren Ende des Flüssigkeitsspeichers 20 schließt sich das Verbindungsstück 30 an, welches sich vorliegend entlang der Mittelachse 2 erstreckt und welches an seinem distalen Ende den Nasalapplikator 40 trägt. Bei anderen Gestaltungen und bevorzugt ist das Verbildungsstück 30 mit einer Biegung oder einen Knick versehen, wie im weiteren noch erläutert wird.

Das Innenvolumen des Hauptkörpers 14 beträgt vorliegend 60 Milliliter, wobei das Innenvolumen des Flüssigkeitsspeichers 20 mehr als 90% des Innenvolumens des Hauptkörpers 14 bildet. Wie sich aus Fig. 1B ersehen lässt, werden vorliegend gut 60% des Innenvolumens des Hauptkörpers 14 von der Flüssigkeit ausgefüllt. Das Gesamtvolumen der Nasenspülflüssigkeit 12 beträgt vorliegend etwa 40 Milliliter. Das Gesamtvolumen ist so zu bemessen, dass die Nasenhöhle des Benutzers mit der Flüssigkeitsmenge einmal vollständig gefüllt werden kann.

Im Lieferzustand ist ein Innenraum 16 des Hauptkörpers 14 vollständig und einstückig vom Hauptkörper 14 umschlossen, so dass Sterilität der Flüssigkeit im Hauptkörper 14 bis zum Zeitpunkt des Öffnens gewährleistet ist. Im Flüssigkeitsspeicher 20 ist somit die Nasenspülflüssigkeit 12 steril gelagert.

Der Nasalapplikator 40 ist dafür ausgebildet, nach Inbetriebnahme durch Entfernen des Verschlusses 50 in ein Nasenloch eines Benutzers eingeführt zu werden und dieses umfänglich abzudichten. Hierfür weist der Nasalapplikator 40 eine in etwa konische oder sich anderweitig zum distalen Ende verjüngende Formgebung auf, die einen Kontaktabschnitt 44 bildet. Dieser ist in der Lage, Nasenlöcher unterschiedlichen Durchmessers abzudichten. Vorliegend beträgt der Außendurchmesser des Nasalapplikators 40 an seiner durchmessergrößten Stelle etwa 1,5 cm. Aufgrund der sich verjüngenden Formgebung kann der Nasalapplikator 40 mit Nasenlochdurchmessern zwischen 0,8 mm und 1,5 mm verwendet werden.

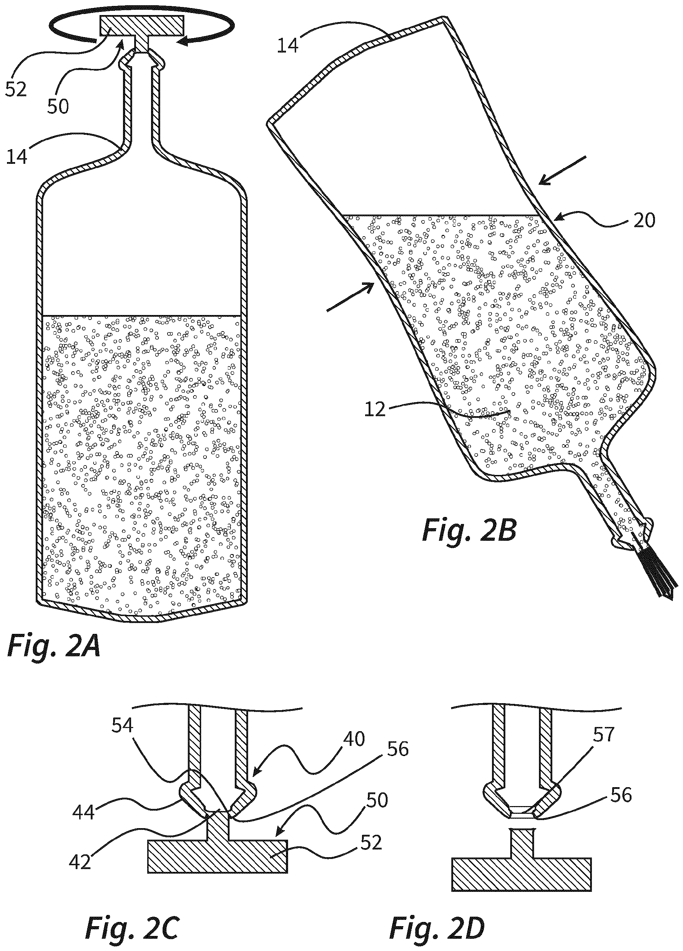

Die Fig. 2A und 2B verdeutlichen die Inbetriebnahme und Nutzung des Nasenspülgeräts 10 durch den Benutzer. Wie in Fig. 2A verdeutlicht, dreht der Benutzer bestimmungsgemäß den Verschluss 50 zunächst ab. Hierfür ist am Verschluss 50 ein flacher und damit gut greifbarer Griffabschnitt 52 vorgesehen, der gegenüber dem restlichen Hauptkörper 14 verdreht wird. Der Verschluss 50 wird hierdurch vom Nasalapplikator 40 getrennt und öffnet dadurch die Abgabeöffnung 42.

Die Fig. 2C und 2D verdeutlichen die Gestaltung des Verschlusses 50. Der Verschluss 50 ist über eine umlaufende Materialbrücke 54 am Nasalapplikator 40 angebracht, die die Trennkante zur Abtrennung des Verschlusses 50 bildet und die gegenüber einem Schutzrand 56 zurückgesetzt ist. Fig. 2D zeigt den Zustand nach dem Abtrennen des Verschlusses. Von der Trennung verbleibt ein Grat 57. Dieser ist jedoch gegenüber dem Schutzrand 56 zurückgesetzt, so dass keine Gefahr besteht, dass der Grat 57 mit Nasenhaut in unmittelbaren und schmerzhaften Kontakt gelangen könnte.

Sobald das Nasenspülgerät 10 nach Entfernen des Verschlusses 50 zur Verwendung bereitsteht, findet die Nutzung statt. Diese erfolgt üblicherweise über einem Waschbecken. Der Benutzer dreht seinen Kopf so, dass der Nasalapplikator 40 in eines seiner Nasenlöcher hineinragt und dieses umlaufend abdichtet. Während des Einführens ist ein Herausfließen der Flüssigkeit nicht zu befürchten, da die lichten Weiten so gewählt sind, dass Luft in dieser Phase nicht durch die Abgabeöffnung 42 austreten kann.

Dies geschieht erst, sobald die Druckbeaufschlagung des Flüssigkeitsspeichers 20 erfolgt, wie in Fig. 2B anhand der auf den Flüssigkeitsspeicher 20 gerichteten Pfeile verdeutlicht ist. Die Wandungen des Hauptkörpers 14 im Bereich des Flüssigkeitsspeichers 20 gestatten ein leichtgängiges elastisches Zusammendrücken, verdeutlicht durch die Pfeile, wobei die Flüssigkeit 12 hierüber dosiert in das Nasenloch abgegeben werden kann. Die Flüssigkeit strömt in die Nasenhöhlen des Benutzers und füllt diese, bis sie anschließend üblicherweise durch das andere Nasenloch wieder austritt.

Üblicherweise wird bei einer Verwendung die gesamte Flüssigkeit abgegeben, gegebenenfalls unter zwischenzeitlichem Wechsel des Nasenlochs.

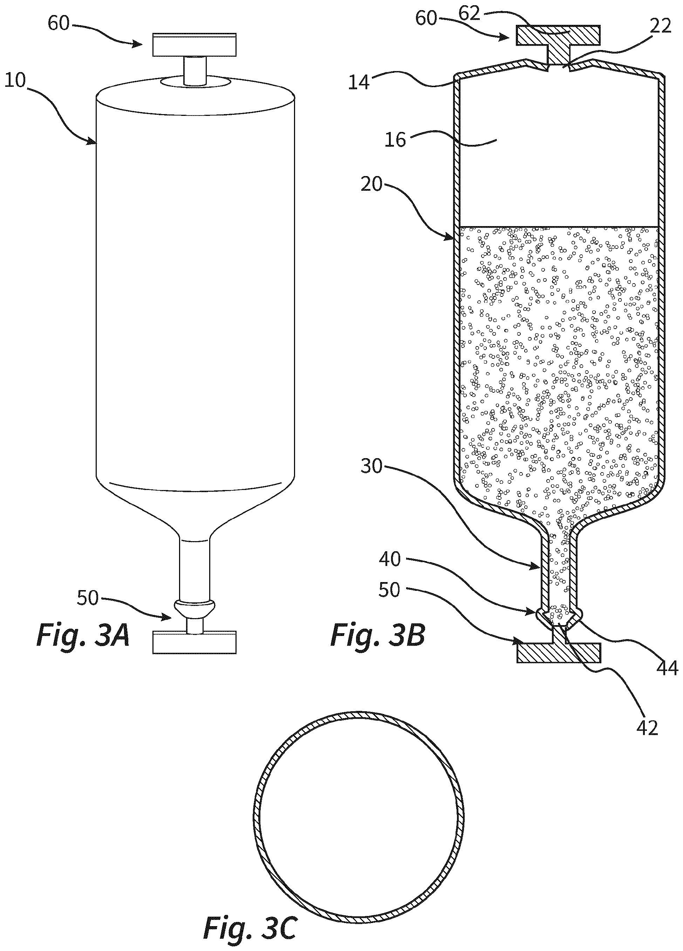

Die Fig. 3A bis 3C zeigen einen zweiten Typ eines Nasenspülgeräts 10. Der wesentliche Unterschied zum ersten Typ besteht darin, dass das Nasenspülgerät 10 der Fig. 3A bis 3C zwei Öffnungen 42, 22 aufweist, die jeweils im Lieferzustand der Fig. 3A bis 3C durch Verschlüsse 50, 60 verschlossen sind.

Übereinstimmend mit der Gestaltung der Fig. 1A bis 2D ist eine der Öffnungen die Abgabeöffnung 42 am Nasalapplikator 40, die mittels des Verschlusses 50 verschlossen ist.

Die andere Öffnung ist eine Lufteinlassöffnung 22, die am gegenüberliegenden Ende des Nasenspülgeräts 10 vorgesehen ist, vorliegend an der gewölbten Wandung, die den Flüssigkeitsspeicher 20 gegenüberliegend zur Abgabeöffnung 42 abschließt. Diese Lufteinlassöffnung 22 ist vom Verschluss 60 verschlossen, der ebenfalls über einen flachen Griffabschnitt 62 verfügt, mittels dessen der Verschluss 60 abgedreht werden kann.

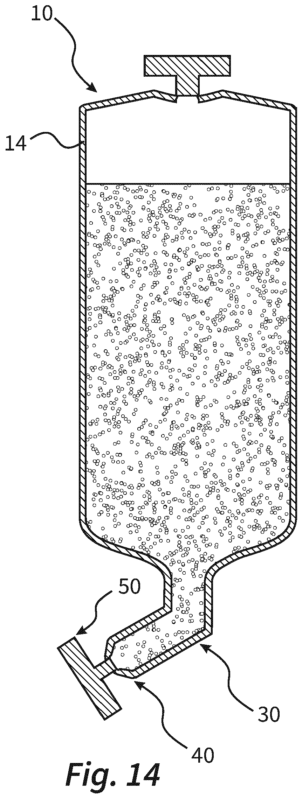

Die Lufteinlassöffnung 22 gestattet bestimmungsgemäß bei der Abgabe von Flüssigkeit das Nachströmen von Luft in den Flüssigkeitsspeicher 20. Abweichend von der Gestaltung der Fig. 1A bis 2D ist somit kein Zusammendrücken des Flüssigkeitsspeichers 20 während der Verwendung erforderlich. Stattdessen strömt die Flüssigkeit aus dem Flüssigkeitsspeicher 20 bei geöffneten Öffnungen 22, 42 schwerkraftbedingt durch die Abgabeöffnung42 aus. Der Verbindungsstück 30 ist bei dieser Ausgestaltung zunächst wie in Fig. 1A dargestellt. Es kann jedoch sinnvoll sein, die Flüssigkeitspfade bei einer schwerkraftbedingten Abgabe weiter zu gestalten, insbesondere in der in Fig. 14 verdeutlichten Weise.

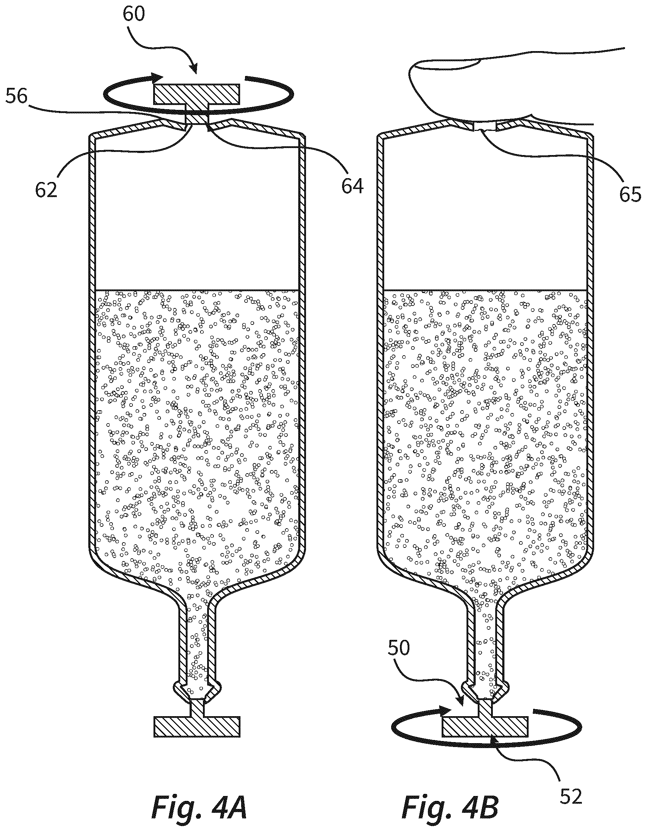

Zur Inbetriebnahme des Nasenspülgeräts 10 der Fig. 3A bis 3C wird zunächst der Verschluss 60 der Lufteinlassöffnung 22 abgedreht, wie in Fig. 4A verdeutlicht. Dies führt aufgrund der noch verschlossenen Abgabeöffnung 42 noch nicht zu einer Flüssigkeitsabgabe.

Zunächst wird die geöffnete Lufteinlassöffnung 22 nochmals wiederum verschlossen, nun mittels eines Fingers des Benutzers, wie Fig. 4B zeigt. Damit der Benutzer nicht in Kontakt mit einem nach der Trennung des Verschlusses 60 verbleibenden Grat 65 gelangt, ist auch die Lufteinlassöffnung 22 von einem Schutzrand 56 umgeben, der die Trennkante umlaufend umgibt und überragt.

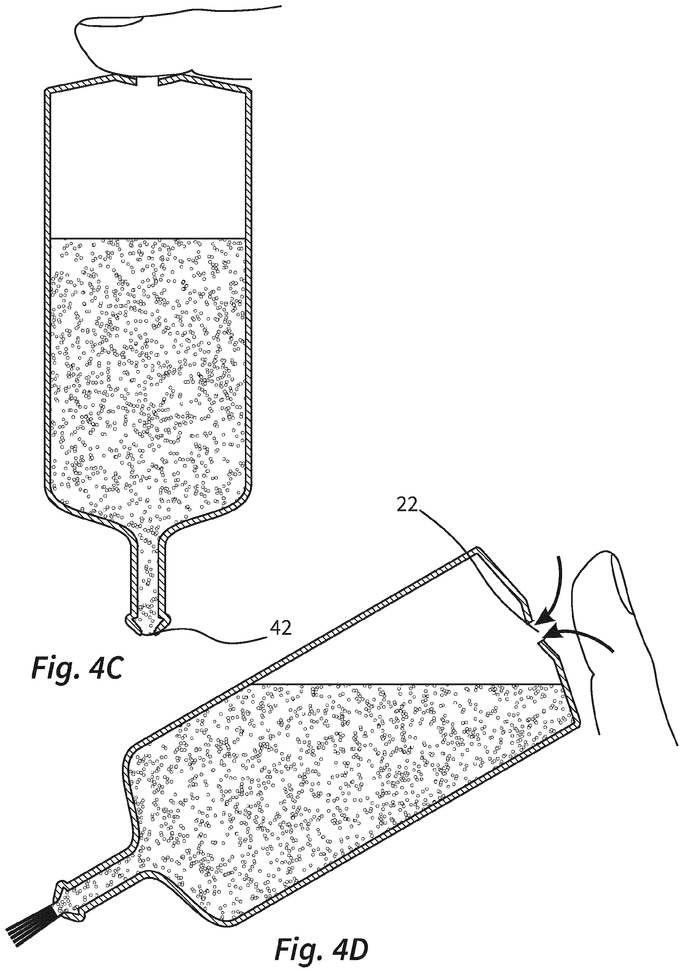

Bei manuell verschlossener Lufteinlassöffnung 22 wird nun der zweite Verschluss 50 der Abgabeöffnung 42 abgetrennt, wie in Fig. 4B verdeutlicht ist. Dies führt noch nicht zum Flüssigkeitsaustrag, wie Fig. 4C zeigt, da keine Luft nachströmen kann. Der Benutzer kann also nun die bestimmungsgemäße Position über einem Waschbecken einnehmen und erst dann den Finger von der Lufteinlassöffnung 22 abheben.

Nun kann in der in Fig. 4D verdeutlichten Weise Flüssigkeit durch die Abgabeöffnung 42 in das Nasenloch des Benutzers ausströmen, während Luft durch die Lufteinlassöffnung 22 nachströmt. Die Pfeile in Fig. 4D verdeutlichen das Nachströmen von Luft.

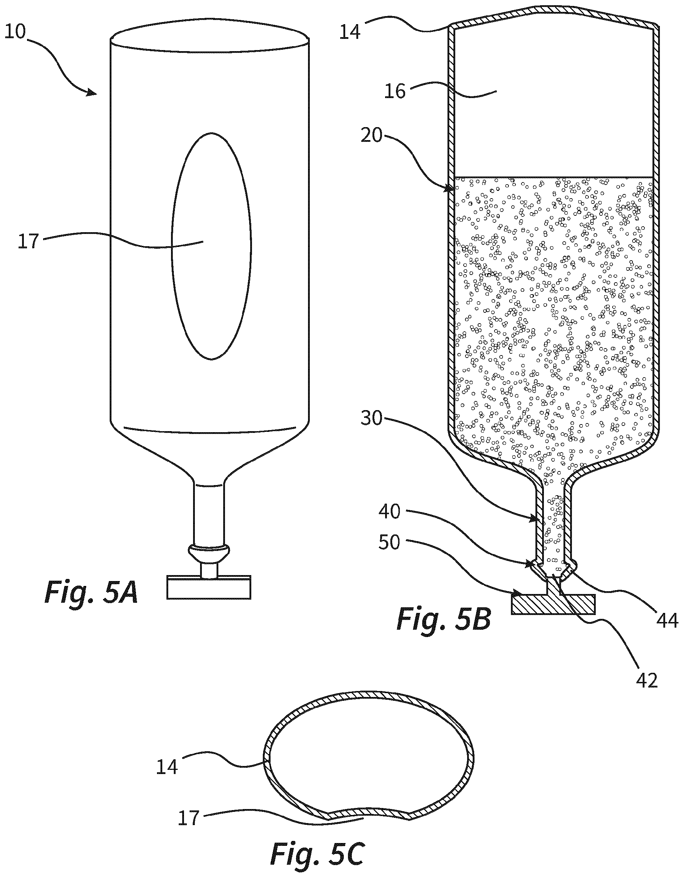

Die Fig. 5A bis 5C zeigen eine weitere Variation eines Nasenspülgeräts 10. Dieses ist entsprechend dem ersten Ausführungsbeispiel Fig. 1A bis 2D nur mit einer Abgabeöffnung 42 und ohne Lufteinlassöffnung 22 gestaltet. Der Flüssigkeitsspeicher 20 ist entsprechend dem ersten Ausführungsbeispiel als Quetsch-Flüssigkeitsspeicher 20 gestaltet, der zum Zwecke des Austrags zusammengedrückt wird. Abweichend hiervon könnte jedoch auch beim Nasenspülgerät 10 der Fig. 5A bis 5C zusätzlich eine im Lieferzustand mit einem Verschluss verschlossene Lufteinlassöffnung 22 vorgesehen sein.

Die Besonderheit der Gestaltung der Fig. 5A bis 5C liegt in der Querschnittsform, wie aus Fig. 5C ersichtlich ist. Der Hauptkörper 14 ist zumindest im Bereich des Flüssigkeitsspeichers 20 mit einem abgeflachten Querschnitt ausgebildet, vorzugsweise mit einem elliptischen Querschnitt. Dies erleichtert die Handhabung, da ein unrunder Querschnitt leichter gefasst und geführt werden kann. Zusätzlich ist beim Ausführungsbeispiel der Fig. 5A bis 5C eine Mulde 17 in einer Vorderwandung des Flüssigkeitsspeichers 20 vorgesehen, um das Zusammendrücken des Flüssigkeitsspeichers 20 zu erleichtern und einem Abrutschen entgegenzuwirken.

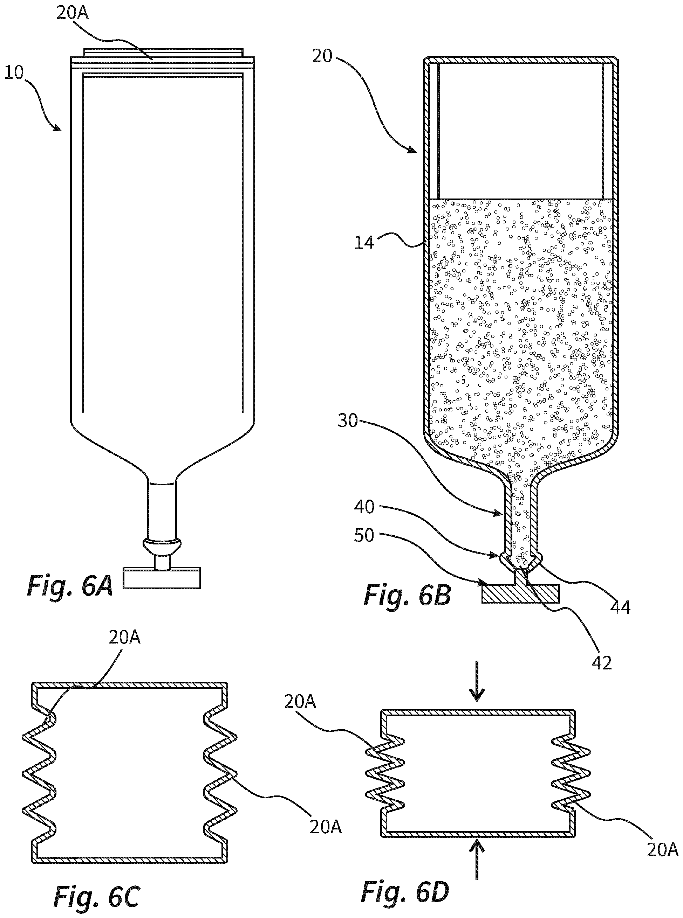

Die Gestaltung der Nasenspülgeräts 10 der Fig. 6A bis 6C ist wiederum eine Gestaltung mit nur einer Abgabeöffnung 42 am Nasalapplikator 40. Wie bei der Gestaltung der Fig. 1A bis 2D ist auch hier vorgesehen, dass nach dem Öffnen der Abgabeöffnung 42 durch Abtrennen des Verschlusses 50 der Austrag durch Zusammendrücken des Flüssigkeitsspeichers 20 erfolgt.

Die Besonderheit bei der Gestaltung der Fig. 6A bis 6C liegt darin, dass der Flüssigkeitsspeicher 20 einen durch Knickverformung entlang von Sollknickstellen oder Biegeverformung entlang von Sollbiegestellen volumenreduzierbaren Flüssigkeitsspeicher aufweist. Wie sich insbesondere der Schnittdarstellung der Fig. 6C entnehmen lässt, weisen zwei Seitenwände 20A des Flüssigkeitsspeichers 20 und dessen Oberseite 20B eine Faltenstruktur auf, die es gestattet, den Flüssigkeitsspeicher 20 mit nur geringer Kraft zusammendrücken zu können. Nach dem Austrag stellt sich ggf. eine elastische Rückstellung ein. Diese ist jedoch nicht zwingend erforderlich.

Die balgartige Gestaltung des Flüssigkeitsspeichers 20 erleichtert die passende Dosierung der Nasenspülflüssigkeit 12, da die Betätigungskraft gering ist. Sie begünstigt darüber hinaus eine platzsparende Konfiguration des Nasenspülgeräts 10 bei dessen Entsorgung.

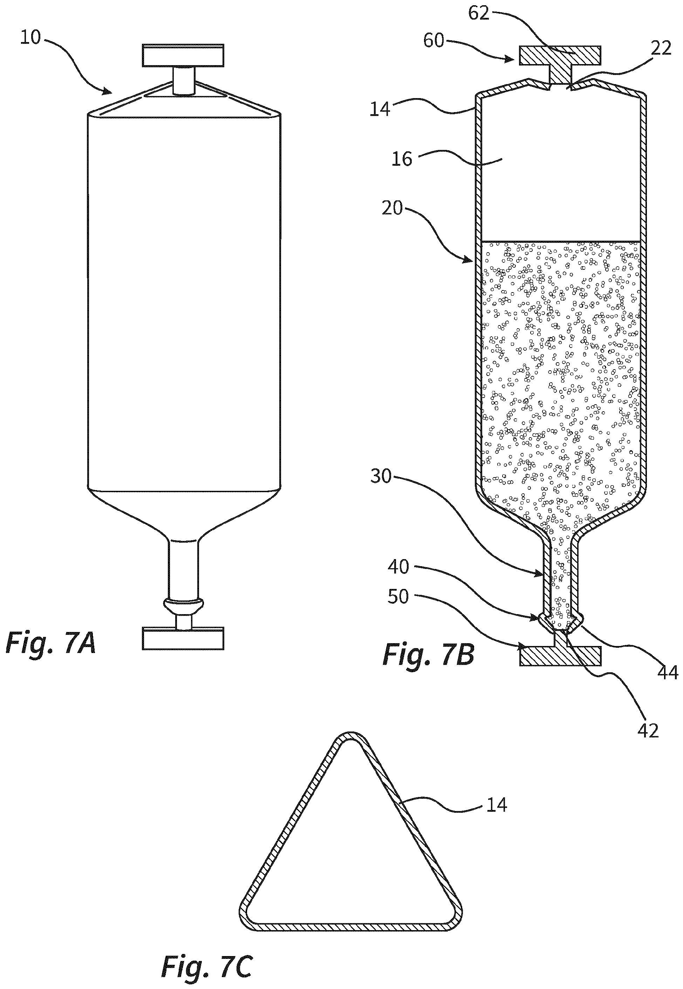

Die Gestaltung der Fig. 7A bis 7C ähnelt wiederum der Gestaltung der Fig. 3A bis 4D. Auch hier ist vorgesehen, dass das Nasenspülgerät 10 über eine Lufteinlassöffnung 22 und eine Abgabeöffnung 42 verfügt, die mit Verschlüssen 50, 60 versehen sind und die nach Entfernung der Verschlüsse ein schwerkraftbedingtes Ausströmen der Flüssigkeit ermöglichen. Auch bei dieser Gestaltung wäre allerdings eine Bauweise mit nur einer Abgabeöffnung 42 und einer bestimmungsgemäßen Nutzung durch Zusammendrücken des Flüssigkeitsspeichers 20 möglich.

Die Besonderheit der Gestaltung der Fig. 7A bis 7C liegt insbesondere in der dreieckigen Querschnittsform, die das Nasenspülgerät 10 am Flüssigkeitsspeicher 20 aufweist, wie insbesondere aus Fig. 7C ersichtlich ist. Solche mehreckigen Gestaltungen, insbesondere mit ausgerundeten Eckbereichen, haben sich bei der Handhabung von Nasenspülgeräten als besonders vorteilhaft herausgestellt. Sie verbessern gegenüber kreiszylindrischen Flüssigkeitsspeichern 20 die Möglichkeit des Benutzers, das Nasenspülgerät 10 richtig auszurichten. Insbesondere bei Gestaltungen mit einem nicht mit dem Flüssigkeitsspeicher 20 fluchtenden Nasalapplikator 40 kann dies einen wesentlichen Vorteil darstellen.

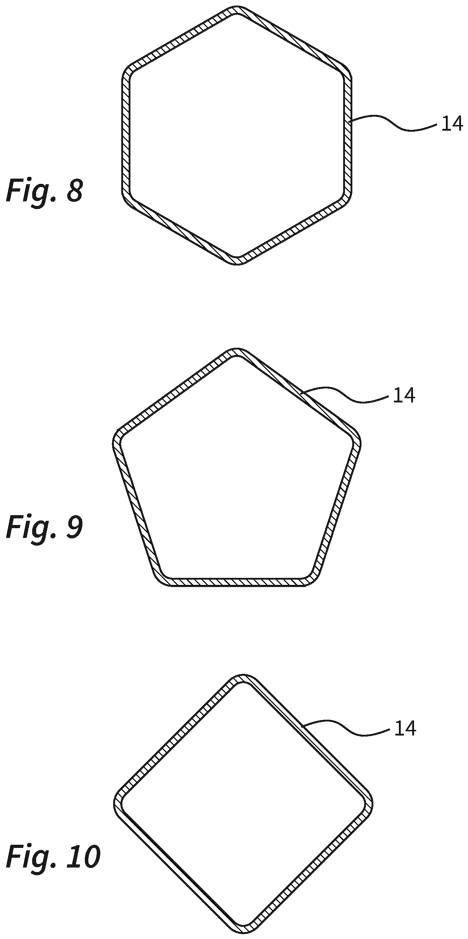

Die dreieckige Gestaltung der Fig. 7C ist nicht alternativlos. Auch viereckige, fünfeckige und sechseckige Gestaltungen des Hauptkörpers 14, insbesondere im Bereich des Flüssigkeitsspeichers, haben sich bewährt. Die entsprechenden Querschnitte sind in den Fig. 8 bis 10 dargestellt.

Die bis hier vorgestellten Gestaltungen hatten gemein, dass der Nasalapplikator 40 parallel zur Richtung der Mittelachse 2 des Flüssigkeitsspeichers 20 ausgerichtet war. Eine solche Bauform kann von Vorteil sein, da sie die Herstellung des Hauptkörpers 14 im Blasform-Verfahren erleichtert.

Als bei der Verwendung vorteilhaft wird es jedoch angesehen, wenn die Ausrichtung des Nasalapplikators 40, insbesondere definiert anhand der Austrittsrichtung von Flüssigkeit, die durch die Abgabeöffnung 42 abgegeben wird, mit der Mittelachse 2 des Flüssigkeitsspeichers 20 einen Winkel einschließt. Insbesondere bei Gestaltungen mit einer Lufteinlassöffnung 22, die zur Abgabe von Flüssigkeit oberhalb der Abgabeöffnung 42 platziert werden muss, ist eine solche Bauweise mit abgeknicktem Nasalapplikator 40 von Vorteil.

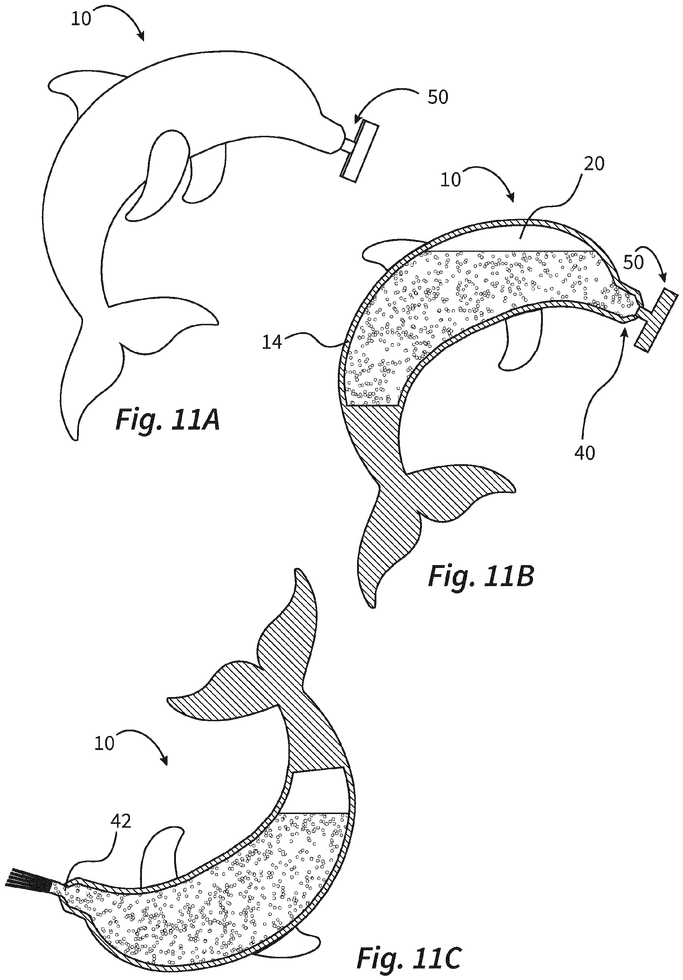

Die Gestaltung der Fig. 11A bis 11C stellt ein Nasenspülgerät 10 dar, welches speziell für Kinder entwickelt worden ist. Dies äußert sich in einer vergleichsweise geringen Flüssigkeitsmenge von 25 ml Nasenspülflüssigkeit in Flüssigkeitsspeicher 20 sowie eine Formgebung des Nasalapplikators 40, der für die Größe von Kinder-Nasenlöchern dimensioniert ist. Die Einstückigkeit des Hauptkörpers 14 des Nasenspülgeräts 10 sowie die einstückige Anformung des Verschlusses 50 ist bei dieser Gestaltung wie auch bei den vorangegangenen Ausführungsbeispielen gestaltet.

Die augenfälligste Besonderheit der Gestaltung der Fig. 11A bis 11C ist die Formgebung des Nasenspülgeräts 10 wie die eines Tieres, vorliegend eines Delphins. Eine solche in Kinderaugen interessante Formgebung steigert die Akzeptanz von Kindern bei der Durchführung einer Nasenspülung.

Bei einer Gestaltung wie der der Fig. 11A bis 11C existiert aufgrund der gekrümmten Formgebung keine definierte Mittelachse des Flüssigkeitsspeichers 20. Dennoch ist gerade diese gekrümmte Form, ob in Tierform oder ohne Tierform, für das Handling von Vorteil. Entsprechend der Ausrichtung der Fig. 11C kann der Spender beim Austrag gehalten werden, so dass das Kind keine unnatürliche Kopfstellung einnehmen muss und die Flüssigkeit dennoch zum überwiegenden Teil oberhalb derAbgabeöffnung42 positioniert ist.

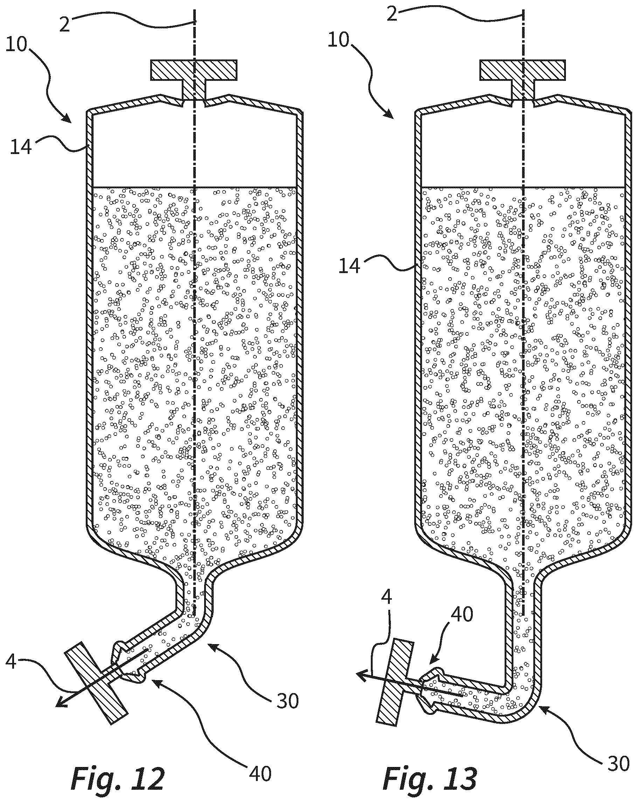

Fig. 12 zeigt eine Gestaltung, bei der der Nasalapplikator 40 gegenüber der Mittelachse 2 des Flüssigkeitsspeichers 20 um etwa 60° angewinkelt ist. Fig. 13 zeigt eine Bauweise, bei der die Anwinkelung etwa 100° entspricht. Wenngleich die meisten der vorgenannten Ausführungsbeispiele mit direkt nach unten weisendem Nasalapplikator 40 ausgerüstet sind, ist eine Bauweise mit einem gegenüber der Mittelachse abgeknickten Nasalapplikator 40 in der Praxis bequemer. Bei allen genannten Gestaltungen der Fig. 1A bis 10 wird eine solche angewinkelte Konfiguration als bevorzugt angesehen.

Die bis hier erläuterten Gestaltungen verfügen über ein verjüngtes Verbindungsstück 30. Eine solche Verjüngung kann als Drossel beim Austrag wirken und so gewählt werden, dass sich ein für die meisten Benutzer angenehmes Gefühl während des Einströmens der Flüssigkeit in die Nasenhöhle einstellt. Ein zu großer Flüssigkeitsstrom wird durch die Drosselwirkung vermieden. Da ein zu großer Flüssigkeitsstrom von Benutzern als sehr unangenehm empfunden wird, ist die genannte Drosselwirkung sehr hilfreich. Dies gilt insbesondere bei Nasenspülgeräten 10, bei die denen der Austrag durch manuelle Druckbeaufschlagung des Flüssigkeitsspeichers 20 erfolgt.

Fig. 14 zeigt eine alternative Gestaltung, bei der zwischen dem Verbindungsstück 30 und dem Nasalapplikator 40 keine Durchmesserstufe mehr vorgesehen ist. Das Verbindungsstück 30 weist dadurch einen großen lichten Querschnitt auf, so dass nur eine geringe Drosselwirkung im Bereich des Verbindungsstücks 30 erzielt wird. Ein solcher Verzicht auf die Drosselwirkung eines gegenüber dem Nasalapplikator 40 verjüngten Verbindungsstücks 30 kann insbesondere dann zweckmäßig sein, wenn der Benutzer über die Lufteinlassöffnung 22 die Flüssigkeitsabgabe fein dosieren kann. In einem solchen Falle ist gewünscht, dass der aufgrund des schwerkraftbedingten Austrags ohnehin geringe Druck in der Flüssigkeit nicht nur eine Drosselwirkung eines Verbindungsstücks 30 weiter eingeschränkt wird.

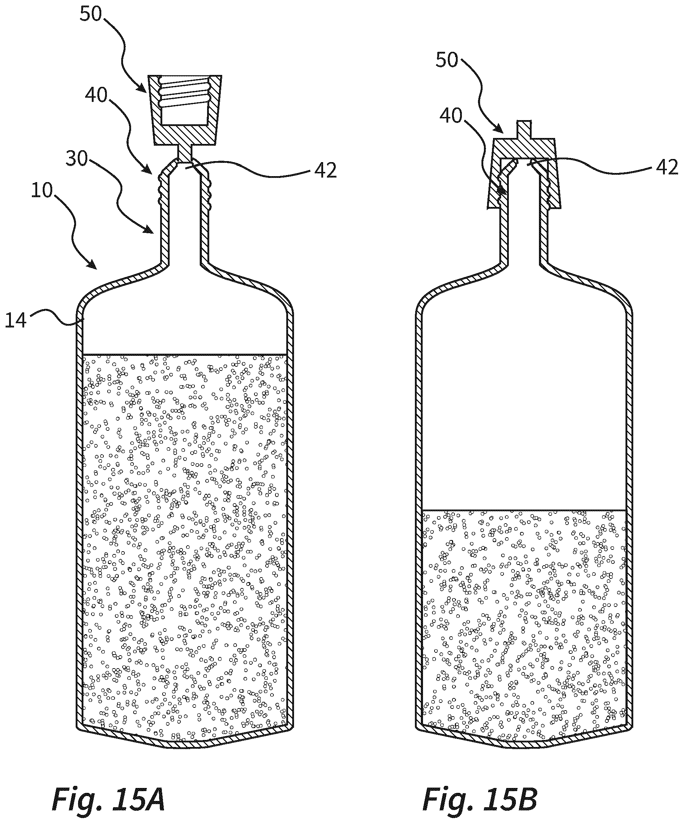

Die Fig. 15A und 15B zeigen eine weitere Gestaltung eines Nasenspülgeräts 10. Die Besonderheit bei dieser Gestaltung liegt in der Wiederverwendbarkeit. Zwar liegt der Schwerpunkt der hier vorgeschlagenen Nasenspülgeräte 10 in der Einweg-Verwendung, also der Entsorgung des Nasenspülgeräts 10 nach einmaliger Verwendung. Es sind jedoch auch Gestaltungen denkbar, die eine Wiederverwendung gestatten, indem der Verschluss 50 an der Abgabeöffnung 42 nach der Trennung eine erneute Befestigung am Nasalapplikator 40 gestattet.

Im Falle der Gestaltung der Fig. 15A und 15B ist vorgesehen, dass am Verbindungsstück 30 ein Außengewinde vorgesehen ist. Korrespondierend hierzu ist der Verschluss 50 anders als bei den vorbeschriebenen Varianten gestaltet, nämlich mit einer Kappenform mit Innengewinde, das im Lieferzustand der Fig. 15A nach außen weist.

Beim Trennen des Verschlusses 50 vom Nasalapplikator 40 wird die Abgabeöffnung 42 in vorbeschriebener Weise geöffnet. Zusätzlich ist es jedoch möglich, den hierbei entfernten Verschluss 50 umzudrehen und dann auf das genannte Außengewinde aufzuschrauben, um hierdurch die Abgabeöffnung 42 wieder zu schließen.

DieFig. 16A bis 16I verdeutlichen einen bevorzugten Herstellungsprozess zur Herstellung eines erfindungsgemäßen Nasenspülgeräts 10, vorliegend exemplarisch des Nasenspülgeräts der Fig. 3A bis 4D.

Die hier vorgeschlagene Art der Herstellung erfolgt mittels sogenanntem Blasformen.

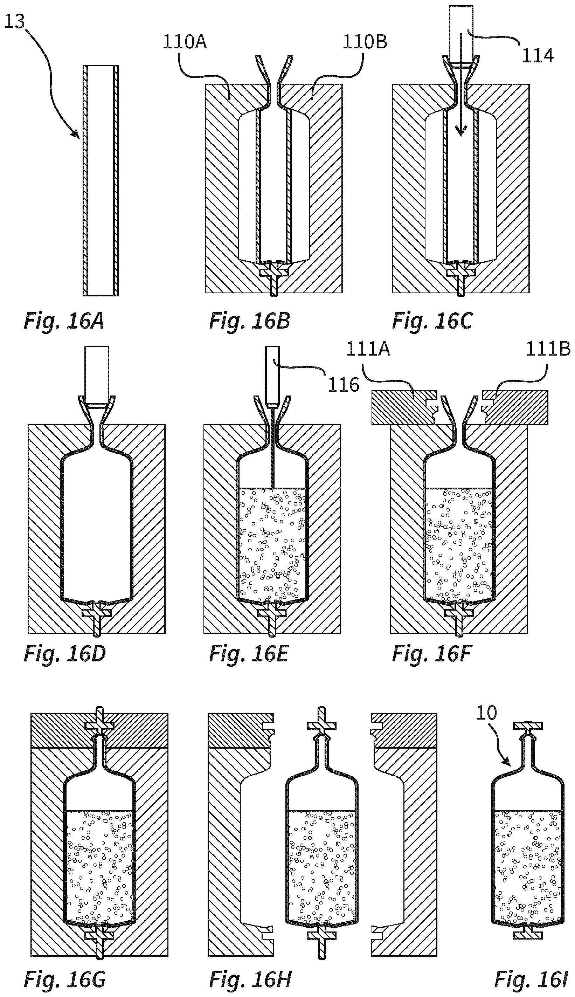

Wie in Fig. 16A dargestellt ist, ist der Ausgangspunkt ein durch Extrusion hergestellter rohrförmiger Rohling 13. Dieser Rohling 13 wird in eine Formkavitäteingesetzt, die aus zwei Teilformen 110A, 110B besteht, und die zuvor bereits erhitzten Teilformen werden in der in Fig. 16B verdeutlichten Weise aufeinander zugefahren, wobei sie hierbei bereits einen Hals des Nasenspülgeräts 10 sowie den Verschluss 60 an der Lufteinlassöffnung 22 bilden.

Anschließend wird, wie in Fig. 16C dargestellt ist, eine Druckluftdüse 114 an das noch offene Ende des bereits teilgeformten Rohlings 13 herangefahren, so dass diese mit dem noch ungeformten oberen Ende des Rohlings 13 abschließt. Anschließend wird Druckluft eingebracht, die den Innenraum des Rohlings 13 unter Druck setzt, so dass sich die Wandungen an die Teilformen 110A, 110B anlegen. Wie in Fig. 16D ersichtlich, erhält der Flüssigkeitsspeicher 20 hierdurch bereits seine Form.

Anschließend erfolgt, dargestellt in Fig. 16E, die Befüllung, wobei der teilgeformte Rohling 13 zu diesem Zeitpunkt noch immer in den Teilformen 110A, 110B angeordnet ist. Es ist jedoch auch ein Prozess denkbar, bei dem der teilgeformte Rohling 13 aus den Teilformen 110A, 110B bereits entnommen ist, bevor das Herstellungsverfahren mit der Befüllung fortgesetzt wird.

Für die Befüllung mit Nasenspülflüssigkeit 12 wird eine Flüssigkeitsdüse 116 oberhalb der Öffnung des teilgeformten Rohlings 13 platziert und hier die zu diesem Zeitpunkt sterile Flüssigkeit abgegeben, so dass sie in den Flüssigkeitsspeicher 20 gelangt.

Sobald die Befüllung mit Nasenspülflüssigkeit 12 gemäß der Darstellung 16E abgeschlossen ist, wird der Rohling 13 bzw. der Hauptkörper 14 im Bereich der noch verbliebenen Öffnung verschlossen. Hierfür sind zwei Siegelformen 111A, 111B vorgesehen, die oberhalb des Halses des teilgeformten Rohling 13 an diesen herangefahren werden und dabei sowohl den Nasalapplikator 40 als auch den hier vorgesehenen Verschluss 50 bilden. Fig. 16F und 16G verdeutlichen dies.

Anschließend werden die Formen 110A, 110B geöffnet, wie in Fig. 16H dargestellt ist und das nahezu fertige Nasenspülgerät 10 kann entnommen werden. Lediglich überschüssiger Kunststoff ist noch zu entfernen, um den Lieferzustand der Fig. 16I herzustellen.

## Patentansprüche

1. Nasenspülgerät (10) mit den folgenden Merkmalen:
a. das Nasenspülgerät (10) weist einen Flüssigkeitsspeicher (20) auf, und
b. das Nasenspülgerät weist einen Nasalapplikator (40) auf, der zur umlaufend abdichtenden Einführung in ein Nasenloch eines Benutzers ausgebildet ist und der der Abgabe der Flüssigkeit aus dem Flüssigkeitsspeicher (20) in ein Nasenloch des Benutzers dient, und
c. der Flüssigkeitsspeicher (20) und der Nasalapplikator (40) sind als starre oder elastisch verformbare Teilkörper ausgebildet und miteinander derart verbunden, dass sie eine im Wesentlichen feste Relativstellung zueinander einnehmen, und
d. am Nasalapplikator (40) ist eine Abgabeöffnung (42) vorgesehen, die zur Abgabe der Flüssigkeit in Form eines unzerstäubten Flüssigkeitsstroms ausgebildet ist,
e. im Nasenspülgerät (10), insbesondere in dessen Flüssigkeitsspeicher (20), ist eine Nasenspülflüssigkeit (12) gelagert, wobei
- das Nasenspülgerät (10) als Kinder-Nasenspülgerät ausgebildet ist und mit einem Flüssigkeitsvolumen von mindestens 20 ml befüllt ist oder
- das Nasenspülgerät (10) als Erwachsenen-Nasenspülgerät ausgebildet ist und mit einem Flüssigkeitsvolumen von mindestens 30 ml befüllt ist,
**gekennzeichnet durch** die folgenden Merkmale:
f. das Nasenspülgerät (10) weist einen einstückigen Hauptkörper (14) auf, der sowohl den Flüssigkeitsspeicher (20) als auch den Nasalapplikator (40) bildet, und
g. der Hauptkörper (14) umschließt im Lieferzustand die Nasenspülflüssigkeit (12) vollumfänglich, wobei die Abgabeöffnung (42) im Lieferzustand durch einen werkzeuglos entfernbaren Verschluss (50) verschlossen ist, der ebenfalls Teil des einstückigen Hauptkörpers (14) ist.

2. Nasenspülgerät (10) nach Anspruch 1 mit den folgenden zusätzlichen Merkmalen:
a. das Nasenspülgerät (10) weist eine Lufteinlassöffnung (22) auf, die während der Abgabe von Flüssigkeit dem Nachströmen von Luft in den Flüssigkeitsspeicher (20) des Nasenspülgeräts dient, und
b. die Lufteinlassöffnung (22) ist im Lieferzustand durch einen werkzeuglos entfernbaren zweiten Verschluss (60) verschlossen, der ebenfalls Teil des einstückigen Hauptkörpers (14) ist.

3. Nasenspülgerät (10) nach Anspruch 1 oder 2 mit dem folgenden zusätzlichen Merkmal:
a. der erste Verschluss (50) und/oder der zweite Verschluss (60) weisen einen Griffabschnitt (52, 62) auf, wobei durch eine Relativbewegung des Verschlusses (50, 60) gegenüber dem Flüssigkeitsspeicher (20) bzw. dem Nasalapplikator (40) der Verschluss werkzeuglos abgetrennt werden kann und hierdurch die Abgabeöffnung (42) oder die Lufteinlassöffnung (22) geöffnet wird.

4. Nasenspülgerät (10) nach einem der vorstehenden Ansprüche mit dem folgenden zusätzlichen Merkmal:
a. der erste Verschluss (50) und/oder der zweite Verschluss (60) sind derart ausgebildet, dass eine Trennkante (54, 64) am Hauptkörper (14), die nach Entfernen des Verschlusses verbleibt, durch einen Schutzrand (56) umgeben ist.

5. Nasenspülgerät (10) nach einem der vorstehenden Ansprüche mit dem folgenden zusätzlichen Merkmal:
a. der Hauptkörper (14) weist mindestens im Bereich des Flüssigkeitsspeichers (20) eine umgebende Wandung (24) auf, deren Dicke zwischen 0,3 mm und 1,0 mm liegt,
vorzugsweise mit mindestens einem der folgenden Merkmale:
b. der Flüssigkeitsspeicher (20) bildet einen elastisch verformbaren Quetschabschnitt (21), der manuell durch den Benutzer volumenreduzierbar ist, wobei die Wandungsstärke der Wandung (24) vorzugsweise zwischen 0,3 mm und 0,6 mm beträgt, oder
c. der Flüssigkeitsspeicher (20) bildet einen starren und bestimmungsgemäß nicht verformbaren Abschnitt, der manuell durch den Benutzer volumenreduzierbar ist, wobei die Wandungsstärke der Wandung (24) vorzugsweise zwischen 0,6 mm und 1,0 mm beträgt.

6. Nasenspülgerät (10) nach einem der vorstehenden Ansprüche mit dem folgenden zusätzlichen Merkmal:
a. der Hauptkörper (14) besteht aus einem der folgenden Materialien:
- Polyethylen,
- Polypropylen,
- Cycloolefin-Copolymere,
- Polyethylenterephthalat,
- Ethyl-Vinylalkohol,
- Polyvinylchlorid, oder
- Polypropylen-Copolymere.

7. Nasenspülgerät (10) nach einem der vorstehenden Ansprüche mit dem folgenden zusätzlichen Merkmal:
a. der Flüssigkeitsspeicher (20) weist eine zylindrische Grundform auf, insbesondere eine kreiszylindrische Grundform,
vorzugsweise mit mindestens einem der folgenden zusätzlichen Merkmale:
b. an einem oberen Ende des Flüssigkeitsspeichers (20) ist die Lufteinlassöffnung (22) vorgesehen, insbesondere fluchtend mit einer Mittelachse (2) der kreiszylindrischen Grundform und/oder
c. an einem unteren Ende des Flüssigkeitsspeichers (20) ist der Nasalapplikator (40) oder ein zum Nasalapplikator (40) führendes Verbindungsstück (30) vorgesehen, insbesondere fluchtend mit einer Mittelachse der kreiszylindrischen Grundform, und/oder
d. der Flüssigkeitsspeicher (20) weist eine kreiszylindrische Grundform mit einem Außendurchmesser zwischen 20 mm und 60 mm auf, vorzugsweise mit einem Außendurchmesser zwischen 25 mm und 40 mm, und/oder
e. der Flüssigkeitsspeicher (20) weist zumindest abschnittsweise einen Querschnitt mit zueinander nicht-parallelen planen Wandungsabschnitten auf, insbesondere einen im Wesentlichen dreieckigen, viereckigen, fünfeckigen oder sechseckigen Querschnitt

8. Nasenspülgerät (10) nach einem der vorstehenden Ansprüche 1 bis 6 mit dem folgenden zusätzlichen Merkmal:
a. das Nasenspülgerät (10) ist als Nasenspülgerät für Kinder ausgebildet und weist die stilisierte Form eines Tieres auf, wobei insbesondere ein Maul, eine Schnauze oder ein Rüssel des stilisierten Tieres den Nasalapplikator (40) bildet,
vorzugsweise mit mindestens einem der folgenden zusätzlichen Merkmale:
b. das Nasenspülgerät (10) weist die stilisierte Form eines Fisches oder eines Delphins auf, oder
c. das Nasenspülgerät (10) weist die stilisierte Form eines Elefanten auf.

9. Nasenspülgerät (10) nach einem der vorstehenden Ansprüche mit dem folgenden zusätzlichen Merkmal:
a. der Flüssigkeitsspeicher (20) weist zumindest abschnittsweise einen flachen Querschnitt auf, der in einer langen Erstreckungsrichtung eine Erstreckung aufweist, die mindestens Faktor 1,5 so groß ist wie in einer kurzen Erstreckungsrichtung,
vorzugsweise mit dem folgenden zusätzlichen Merkmal:
b. der Flüssigkeitsspeicher (20) weist zumindest abschnittsweise einen elliptischen Querschnitt auf.

10. Nasenspülgerät (10) nach einem der vorstehenden Ansprüche mit dem folgenden zusätzlichen Merkmal:
a. der Flüssigkeitsspeicher (20) ist zumindest abschnittsweise mit einem Balgabschnitt ausgebildet, der die Reduzierung des Innenvolumens erleichtert.

11. Nasenspülgerät (10) nach einem der vorstehenden Ansprüche mit dem folgenden zusätzlichen Merkmal:
a. der Flüssigkeitsspeicher (20) ist als Quetschspeicher ausgebildet und weist hierfür zwei einander gegenüberliegende Betätigungs-Wandabschnitte zum Zwecke der manuellen Krafteinkopplung auf,
vorzugsweise mit dem folgenden zusätzlichen Merkmal:
b. an mindestens einem der Betätigungs-Wandabschnitte ist eine vertiefte oder mit Greifstrukturierungen versehene Grifffläche (17) vorgesehen.

12. Nasenspülgerät (10) nach einem der vorstehenden Ansprüche mit den folgenden zusätzlichen Merkmalen:
a. der Flüssigkeitsspeicher (20) und der Nasalapplikator (40) sind über ein Verbindungsstück (30) miteinander verbunden, und
b. das Verbindungsstück (30) bewirkt eine Anwinkelung des Nasalapplikators (40) vom Flüssigkeitsspeicher (20), wobei insbesondere ein Winkel zwischen einer Mittelachse (2) der kreiszylindrischen Grundform und der durch die Abgabeöffnung (42) definierten Abgaberichtung (4) zwischen 60° und 120° beträgt,
vorzugsweise mit einem der folgenden zusätzlichen Merkmale:
c. das Verbindungsstück (30) weist einen Außendurchmesser auf, der dem größten Außendurchmesser am Nasalapplikator (40) entspricht oder diesen übersteigt, oder
d. das Verbindungsstück (30) weist einen Außendurchmesser auf, der kleiner ist als der größte Außendurchmesser am Nasalapplikator (40).

13. Nasenspülgerät nach einem der vorstehenden Ansprüche mit dem folgenden zusätzlichen Merkmal:
a. der Nasalapplikator (40) weist einen konischen Kontaktabschnitt (44) zur Anlage an einer Nasenlochwandungdes Benutzers auf, wobei der konische Kontaktabschnitt (44) an seiner durchmessergrößten Stelle einen Außendurchmesser von mindestens 15 mm aufweist,
vorzugsweise mit dem folgenden zusätzlichen Merkmal:
b. der Nasalapplikator (40) ist am Ende eines Verbindungsstücks (30) vorgesehen, dessen Außendurchmesser geringer ist als der Außendurchmesser des Kontaktabschnitts (44) an seiner durchmessergrößten Stelle.

14. Nasenspülgerät (10) nach einem der vorstehenden Ansprüche mit dem folgenden zusätzlichen Merkmal:
a. ein Innenvolumen des Hauptkörpers (14) ist zu maximal 90% mit der Nasenspülflüssigkeit (12) befüllt, vorzugsweise zu maximal 75%,
vorzugsweise mit dem folgenden zusätzlichen Merkmal:
b. der dem Austrag der Flüssigkeit ist der Flüssigkeitsspiegel bei tief angeordnetem Nasalapplikator (40) vorzugsweise mindestens 80 mm oberhalb der Abgabeöffnung (42), vorzugsweise mindestens 100 mm oberhalb der Abgabeöffnung (42).

15. Nasenspülgerät (10) nach einem der vorstehenden Ansprüche mit dem folgenden zusätzlichen Merkmal:
a. die Abgabeöffnung (42) weist eine lichte Durchtrittsfläche von mindestens 3 mm² auf, insbesondere vorzugsweise von mindestens 6 mm² oder mindestens 10 mm²,
vorzugsweise mit dem folgenden zusätzlichen Merkmal:
b. ein Flüssigkeitskanal vom Flüssigkeitsspeicher (20) zur Abgabeöffnung (42) weist einen geringsten lichten Querschnitt von mindestens 3 mm² auf, insbesondere vorzugsweise von mindestens 6 mm² oder mindestens 10 mm².

16. Nasenspülgerät (10) nach einem der vorstehenden Ansprüche mit mindestens einem der folgenden zusätzlichen Merkmale:
a. im Nasenspülgerät (10), insbesondere in dessen Flüssigkeitsspeicher (20), ist eine Nasenspülflüssigkeit (12) mit einem Flüssigkeitsvolumen von mindestens 40 ml gelagert, vorzugsweise von mindestens 50 ml, und/oder
b. die Nasenspülflüssigkeit (12) ist eine konservierungsmittelfreie Nasenspülflüssigkeit, und/oder
c. die Verschlüsse (50, 60) sind als wiederverwendbare Verschlüsse ausgebildet, und/oder
d. der Hauptkörper (14) ist vollständig oder abschnittsweise transparent ausgebildet, um die im Hauptkörper (14) verbleibende Flüssigkeitsmenge für den Benutzer erkennbar zu machen.

17. Verfahren zur Herstellung eines Nasenspülgeräts (10) nach einem der vorstehenden Ansprüche mit den folgenden Schritten:
a. der Hauptkörper (14) des Nasenspülgeräts (10), der sowohl den Flüssigkeitsspeicher (20) als auch den Nasalapplikator (40) bildet, wird in einer Formkavität als einstückige Einheit geformt, wobei zunächst eine Öffnung im Hauptkörper (14) verbleibt, und
b. nach der Formung des Hauptkörpers (14) wird die Nasenspülflüssigkeit (12) in einem sterilen Zustand durch die Öffnung in den Flüssigkeitsspeicher (20) des Hauptkörpers (14) eingefüllt, ohne dass der Hauptkörper (14) hierfür die sterile Umgebung verlässt, und
c. nach Einfüllung der Nasenspülflüssigkeit (12) wird der Hauptkörper (14) unter Nutzung des Materials des Hauptkörpers (14) verschlossen, vorzugsweise thermisch verschlossen,
vorzugsweise mit den folgenden zusätzlichen Merkmalen
d. zur Herstellung der Hauptkörpers (14) wird zunächst mittels Extrusion ein Schlauchkörper hergestellt, und
e. der Hauptkörper (14) wird ausgehend von diesem Schlauchkörper geformt, indem der Schlauchkörper oder ein Teilabschnitt des Schlauchkörpers in der Formkavität aufgeblasen wird, so dass er den zu diesem Zeitpunkt noch mit einer Öffnung versehenen Hauptkörper (14) bildet.
